(19) Europäisches Patentamt
European Patent Office
Office européen des brevets

(11) EP 3 854 815 A1

(12) **EUROPEAN PATENT APPLICATION**
published in accordance with Art. 153(4) EPC

(43) Date of publication:
**28.07.2021 Bulletin 2021/30**

(21) Application number: **18936571.1**

(22) Date of filing: **10.10.2018**

(51) Int Cl.:
***C07K 16/28*** *(2006.01)*
***C12N 15/62*** *(2006.01)*
***C12N 5/10*** *(2006.01)*
***A61K 35/17*** *(2015.01)*
***A61P 35/00*** *(2006.01)*

(86) International application number:
**PCT/CN2018/109564**

(87) International publication number:
**WO 2020/073215 (16.04.2020 Gazette 2020/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **BGI Shenzhen**
**Shenzhen, Guangdong 518083 (CN)**

(72) Inventors:
- **WANG, Meiniang**
  **Shenzhen, Guangdong 518083 (CN)**
- **OUYANG, Bingjie**
  **Shenzhen, Guangdong 518083 (CN)**
- **YANG, Naibo**
  **Shenzhen, Guangdong 518083 (CN)**
- **JIANG, Lin**
  **Shenzhen, Guangdong 518083 (CN)**
- **REN, Bingzhao**
  **Shenzhen, Guangdong 518083 (CN)**
- **LIU, Yang**
  **Shenzhen, Guangdong 518083 (CN)**
- **ZHAO, Zhengqi**
  **Shenzhen, Guangdong 518083 (CN)**
- **LI, Bo**
  **Shenzhen, Guangdong 518083 (CN)**
- **HOU, Yong**
  **Shenzhen, Guangdong 518083 (CN)**
- **WANG, Fei**
  **Shenzhen, Guangdong 518083 (CN)**
- **GE, Yuping**
  **Shenzhen, Guangdong 518083 (CN)**
- **DONG, Xuan**
  **Shenzhen, Guangdong 518083 (CN)**

(74) Representative: **Biggi, Cristina**
**Bugnion S.p.A.**
**Viale Lancetti 17**
**20158 Milano (IT)**

(54) **ANTI-BCMA SINGLE-CHAIN ANTIBODY SCFV, PREPARATION METHOD THEREFOR AND APPLICATION THEREOF**

(57) Provided are an anti-BCMA single-chain antibody scFv and a preparation method and application thereof. The preparation method is capable of artificially synthesizing a BCMA-targeted phage display library by combining site-directed mutagenesis and random mutagenesis technologies; and acquiring 3 new-type BCMA antigen-targeted scFv strains to serve as BCMA-targeted antibodies by combining a phage display technology.

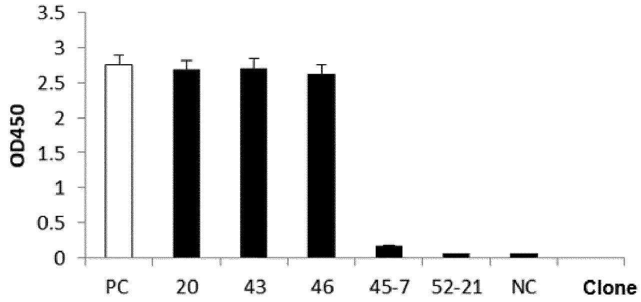


Fig. 5

EP 3 854 815 A1

Printed by Jouve, 75001 PARIS (FR)

SEQUENCE LISTING

<110> BGI SHENZHEN

<120> Anti-BCMA single chain antibody scFv, preparation method thereof and application thereof

<130> PN151335HDZZ

<160> 65

<170> PatentIn version 3.5

<210> 1
<211> 249
<212> PRT
<213> Homo sapiens

<400> 1

```
Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Asn Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Arg Lys Leu Ala Trp
                85                  90                  95

Thr Phe Gly Gln Gly Thr Lys Leu Glu Ile Lys Arg Gly Gly Gly Gly
            100                 105                 110

Ser Gly Gly Gly Gly Ser Gly Gly Gly Gly Ser Gly Gly Gly Gly Ser
        115                 120                 125

Gln Val Gln Leu Val Gln Ser Gly Ala Glu Val Lys Lys Pro Gly Ser
    130                 135                 140

Ser Val Lys Val Ser Cys Lys Ala Ser Gly Gly Thr Phe Ser Asn Tyr
145                 150                 155                 160

Trp Met His Trp Val Arg Gln Ala Pro Gly Gln Gly Leu Glu Trp Met
                165                 170                 175
```

```
Gly Ala Thr Tyr Arg Gly His Ser Asp Thr Tyr Tyr Asn Gln Lys Phe
            180                 185                 190

Lys Gly Arg Val Thr Ile Thr Ala Asp Lys Ser Thr Ser Thr Ala Tyr
        195                 200                 205

Met Glu Leu Ser Ser Leu Arg Ser Glu Asp Thr Ala Val Tyr Tyr Cys
    210                 215                 220

Ala Arg Gly Ser Ile Phe Asn Gly Tyr Asp Val Leu Asp Asn Trp Gly
225                 230                 235                 240

Gln Gly Thr Leu Val Thr Val Ser Ser
                245
```

```
<210>  2
<211>  747
<212>  DNA
<213>  Homo sapiens

<400>  2
gacatccaga tgacccagag ccctagctca ctgagcgcca gcgtgggcga cagggtgacc      60

attacctgct ccgccagcca ggacatcagc aactacctga actggtacca gcagaagccc     120

ggcaaggccc ccaagctgct gatctactac acctccaacc tgcactccgg cgtgcccagc     180

aggttcagcg gaagcggcag cggcaccgat ttcaccctga ccatctccag cctgcagccc     240

gaggacttcg ccacctacta ctgccagcag tacaggaagc tcgcatggac tttcggccag     300

ggcaccaaac tggagatcaa gcgtggtgga ggaggtagcg gaggaggcgg gagcggtgga     360

ggtggctctg gaggtggcgg aagccaggtg cagctggtcc agagcggcgc cgaagtgaag     420

aagcccggca gctccgtgaa agtgagctgc aaggccagcg gcggcacctt cagcaactac     480

tggatgcact gggtgaggca ggcccccgga cagggcctgg agtggatggg cgccacctac     540

aggggccaca gcgacaccta ctacaaccag aagttcaagg gccgggtgac catcaccgcc     600

gacaagagca ccagcaccgc ctacatggaa ctgagcagcc tcaggagcga ggacaccgct     660

gtgtattact gcgccagggg ttctattttc aacggttacg acgttctgga caactggggc     720

cagggcacac tagtgaccgt gtccagc                                         747
```

```
<210>  3
<211>  121
<212>  PRT
<213>  Homo sapiens

<400>  3

Gln Val Gln Leu Val Gln Ser Gly Ala Glu Val Lys Lys Pro Gly Ser
1               5                   10                  15
```

```
Ser Val Lys Val Ser Cys Lys Ala Ser Gly Gly Thr Phe Ser Asn Tyr
            20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Ser Ile Phe Asn Gly Tyr Asp Val Leu Asp Asn Trp Gly
            100                 105                 110

Gln Gly Thr Leu Val Thr Val Ser Ser
        115                 120


<210>  4
<211>  108
<212>  PRT
<213>  Homo sapiens

<400>  4

Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Asn Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Arg Lys Leu Ala Trp
                85                  90                  95

Thr Phe Gly Gln Gly Thr Lys Leu Glu Ile Lys Arg
            100                 105
```

```
<210>  5
<211>  244
<212>  PRT
<213>  Homo sapiens

<400>  5

Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Asn Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ala Leu Val Val Thr Pro
                85                  90                  95

Phe Thr Phe Gly Gln Gly Thr Lys Leu Glu Ile Lys Arg Gly Gly Gly
            100                 105                 110

Gly Ser Gly Gly Gly Gly Ser Gly Gly Gly Gly Ser Gly Gly Gly Gly
        115                 120                 125

Ser Gln Val Gln Leu Val Gln Ser Gly Ala Glu Val Lys Lys Pro Gly
    130                 135                 140

Ser Ser Val Lys Val Ser Cys Lys Ala Ser Gly Gly Thr Phe Ser Asn
145                 150                 155                 160

Tyr Trp Met His Trp Val Arg Gln Ala Pro Gly Gln Gly Leu Glu Trp
                165                 170                 175

Met Gly Ala Thr Tyr Arg Gly His Ser Asp Thr Tyr Tyr Asn Gln Lys
            180                 185                 190

Phe Lys Gly Arg Val Thr Ile Thr Ala Asp Lys Ser Thr Ser Thr Ala
        195                 200                 205

Tyr Met Glu Leu Ser Ser Leu Arg Ser Glu Asp Thr Ala Val Tyr Tyr
    210                 215                 220
```

Cys Ala Arg Phe Gly Met Leu Asp Asn Trp Gly Gln Gly Thr Leu Val
225 230 235 240

Thr Val Ser Ser

<210> 6
<211> 735
<212> DNA
<213> Homo sapiens

<400> 6
gacatccaga tgacccagag ccctagctca ctgagcgcca gcgtgggcga cagggtgacc 60

attacctgct ccgccagcca ggacatcagc aactacctga actggtacca gcagaagccc 120

ggcaaggccc ccaagctgct gatctactac acctccaacc tgcactccgg cgtgcccagc 180

aggttcagcg gaagcggcag cggcaccgat ttcaccctga ccatctccag cctgcagccc 240

gaggacttcg ccacctacta ctgccagcag gctcttgtgg tgacgccgtt cactttcggc 300

cagggcacca aactggagat caagcgtggt ggaggaggta gcggaggagg cgggagcggt 360

ggaggtggct ctggaggtgg cggaagccag gtgcagctgg tccagagcgg cgccgaagtg 420

aagaagcccg gcagctccgt gaaagtgagc tgcaaggcca gcggcggcac cttcagcaac 480

tactggatgc actgggtgag gcaggccccc ggacagggcc tggagtggat gggcgccacc 540

tacaggggcc acagcgacac ctactacaac cagaagttca agggccgggt gaccatcacc 600

gccgacaaga gcaccagcac cgcctacatg gaactgagca gcctcaggag cgaggacacc 660

gctgtgtatt actgcgccag gttttagggt atgctggaca actggggcca gggcacacta 720

gtgaccgtgt ccagc 735

<210> 7
<211> 115
<212> PRT
<213> Homo sapiens

<400> 7

Gln Val Gln Leu Val Gln Ser Gly Ala Glu Val Lys Lys Pro Gly Ser
1 5 10 15

Ser Val Lys Val Ser Cys Lys Ala Ser Gly Gly Thr Phe Ser Asn Tyr
20 25 30

Trp Met His Trp Val Arg Gln Ala Pro Gly Gln Gly Leu Glu Trp Met
35 40 45

Gly Ala Thr Tyr Arg Gly His Ser Asp Thr Tyr Tyr Asn Gln Lys Phe

50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Phe Gly Met Leu Asp Asn Trp Gly Gln Gly Thr Leu Val Thr
100 105 110

Val Ser Ser
115

<210> 8
<211> 109
<212> PRT
<213> Homo sapiens

<400> 8

Asp Ile Gln Met Thr Gln Ser Pro Ser Ser Leu Ser Ala Ser Val Gly
1 5 10 15

Asp Arg Val Thr Ile Thr Cys Ser Ala Ser Gln Asp Ile Ser Asn Tyr
20 25 30

Leu Asn Trp Tyr Gln Gln Lys Pro Gly Lys Ala Pro Lys Leu Leu Ile
35 40 45

Tyr Tyr Thr Ser Asn Leu His Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Ala Leu Val Val Thr Pro
85 90 95

Phe Thr Phe Gly Gln Gly Thr Lys Leu Glu Ile Lys Arg
100 105

<210> 9
<211> 245
<212> PRT
<213> Homo sapiens

<400> 9

Asp Ile Gln Met Thr Gln Ser Pro Ser Ser Leu Ser Ala Ser Val Gly
1 5 10 15

Asp Arg Val Thr Ile Thr Cys Ser Ala Ser Gln Asp Ile Ser Asn Tyr
20 25 30

Leu Asn Trp Tyr Gln Gln Lys Pro Gly Lys Ala Pro Lys Leu Leu Ile
35 40 45

Tyr Tyr Thr Ser Asn Leu His Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Arg Leu Thr Pro Ser Pro
85 90 95

Phe Thr Phe Gly Gln Gly Thr Lys Leu Glu Ile Lys Arg Gly Gly Gly
100 105 110

Gly Ser Gly Gly Gly Gly Ser Gly Gly Gly Gly Ser Gly Gly Gly Gly
115 120 125

Ser Gln Val Gln Leu Val Gln Ser Gly Ala Glu Val Lys Lys Pro Gly
130 135 140

Ser Ser Val Lys Val Ser Cys Lys Ala Ser Gly Gly Thr Phe Ser Asn
145 150 155 160

Tyr Trp Met His Trp Val Arg Gln Ala Pro Gly Gln Gly Leu Glu Trp
165 170 175

Met Gly Ala Thr Tyr Arg Gly His Ser Asp Thr Tyr Tyr Asn Gln Lys
180 185 190

Phe Lys Gly Arg Val Thr Ile Thr Ala Asp Lys Ser Thr Ser Thr Ala
195 200 205

Tyr Met Glu Leu Ser Ser Leu Arg Ser Glu Asp Thr Ala Val Tyr Tyr
210 215 220

Cys Ala Arg Asn Thr Ala Leu Leu Asp Asn Trp Gly Gln Gly Thr Leu
225 230 235 240

Val Thr Val Ser Ser
245

<210> 10

<211> 735
<212> DNA
<213> Homo sapiens

<400> 10
gacatccaga tgacccagag ccctagctca ctgagcgcca gcgtgggcga cagggtgacc 60

attacctgct ccgccagcca ggacatcagc aactacctga actggtacca gcagaagccc 120

ggcaaggccc ccaagctgct gatctactac acctccaacc tgcactccgg cgtgcccagc 180

aggttcagcg gaagcggcag cggcaccgat ttcaccctga ccatctccag cctgcagccc 240

gaggacttcg ccacctacta ctgccagcag cgtttgacgc cgtctccgtt cactttcggc 300

cagggcacca aactggagat caagcgtggt ggaggaggta gcggaggagg cgggagcggt 360

ggaggtggct ctggaggtgg cggaagccag gtgcagctgg tccagagcgg cgccgaagtg 420

aagaagcccg gcagctccgt gaaagtgagc tgcaaggcca gcggcggcac cttcagcaac 480

tactggatgc actgggtgag gcaggccccc ggacagggcc tggagtggat gggcgccacc 540

tacaggggcc acagcgacac ctactacaac cagaagttca agggccgggt gaccatcacc 600

gccgacaaga gcaccagcac cgcctacatg gaactgagca gcctcaggag cgaggacacc 660

gctgtgtatt actgcgccag gaatacggct ttgctggaca actggggcca gggcacacta 720

gtgaccgtgt ccagc 735

<210> 11
<211> 116
<212> PRT
<213> Homo sapiens

<400> 11

Gln Val Gln Leu Val Gln Ser Gly Ala Glu Val Lys Lys Pro Gly Ser
1 5 10 15

Ser Val Lys Val Ser Cys Lys Ala Ser Gly Gly Thr Phe Ser Asn Tyr
20 25 30

Trp Met His Trp Val Arg Gln Ala Pro Gly Gln Gly Leu Glu Trp Met
35 40 45

Gly Ala Thr Tyr Arg Gly His Ser Asp Thr Tyr Tyr Asn Gln Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Thr Ala Leu Leu Asp Asn Trp Gly Gln Gly Thr Leu Val
100 105 110

Thr Val Ser Ser
115

<210> 12
<211> 108
<212> PRT
<213> Homo sapiens

<400> 12

Asp Ile Gln Met Thr Gln Ser Pro Ser Ser Leu Ser Ala Ser Val Gly
1 5 10 15

Asp Arg Val Thr Ile Thr Cys Ser Ala Ser Gln Asp Ile Ser Asn Tyr
20 25 30

Leu Asn Trp Tyr Gln Gln Lys Pro Gly Lys Ala Pro Lys Leu Leu Ile
35 40 45

Tyr Tyr Thr Ser Asn Leu His Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Arg Leu Thr Pro Ser Pro
85 90 95

Phe Thr Phe Gly Gln Gly Thr Lys Leu Glu Ile Lys
100 105

<210> 13
<211> 26
<212> PRT
<213> Homo sapiens

<400> 13

Asp Ile Gln Met Thr Gln Ser Pro Ser Ser Leu Ser Ala Ser Val Gly
1 5 10 15

Asp Arg Val Thr Ile Thr Cys Ser Ala Ser
20 25

<210> 14
<211> 6
<212> PRT
<213> Homo sapiens

<400> 14

Gln Asp Ile Ser Asn Tyr
1               5

<210> 15
<211> 17
<212> PRT
<213> Homo sapiens

<400> 15

Leu Asn Trp Tyr Gln Gln Lys Pro Gly Lys Ala Pro Lys Leu Leu Ile
1               5                   10                  15

Tyr

<210> 16
<211> 4
<212> PRT
<213> Homo sapiens

<220>
<221> MISC_FEATURE
<222> (4)..(4)
<223> Xaa is not existed, just for finishing the sequence listing

<400> 16

Tyr Thr Ser Xaa
1

<210> 17
<211> 36
<212> PRT
<213> Homo sapiens

<400> 17

Asn Leu His Ser Gly Val Pro Ser Arg Phe Ser Gly Ser Gly Ser Gly
1               5                   10                  15

Thr Asp Phe Thr Leu Thr Ile Ser Ser Leu Gln Pro Glu Asp Phe Ala
            20                  25                  30

Thr Tyr Tyr Cys
        35

<210> 18
<211> 9
<212> PRT
<213> Homo sapiens

<400> 18

Gln Gln Tyr Arg Lys Leu Ala Trp Thr
1               5

<210>  19
<211>  11
<212>  PRT
<213>  Homo sapiens

<400>  19

Phe Gly Gln Gly Thr Lys Leu Glu Ile Lys Arg
1               5                   10

<210>  20
<211>  25
<212>  PRT
<213>  Homo sapiens

<400>  20

Gln Val Gln Leu Val Gln Ser Gly Ala Glu Val Lys Lys Pro Gly Ser
1               5                   10                  15

Ser Val Lys Val Ser Cys Lys Ala Ser
            20                  25

<210>  21
<211>  8
<212>  PRT
<213>  Homo sapiens

<400>  21

Gly Gly Thr Phe Ser Asn Tyr Trp
1               5

<210>  22
<211>  17
<212>  PRT
<213>  Homo sapiens

<400>  22

Met His Trp Val Arg Gln Ala Pro Gly Gln Gly Leu Glu Trp Met Gly
1               5                   10                  15

Ala

<210>  23
<211>  8
<212>  PRT
<213>  Homo sapiens

<400>  23

```
Thr Tyr Arg Gly His Ser Asp Thr
1               5
```

```
<210>  24
<211>  38
<212>  PRT
<213>  Homo sapiens

<400>  24

Tyr Tyr Asn Gln Lys Phe Lys Gly Arg Val Thr Ile Thr Ala Asp Lys
1               5                   10                  15

Ser Thr Ser Thr Ala Tyr Met Glu Leu Ser Ser Leu Arg Ser Glu Asp
            20                  25                  30

Thr Ala Val Tyr Tyr Cys
        35
```

```
<210>  25
<211>  14
<212>  PRT
<213>  Homo sapiens

<400>  25

Ala Arg Gly Ala Ile Tyr Asn Gly Tyr Asp Val Leu Asp Asn
1               5                   10
```

```
<210>  26
<211>  11
<212>  PRT
<213>  Homo sapiens

<400>  26

Trp Gly Gln Gly Thr Leu Val Thr Val Ser Ser
1               5                   10
```

```
<210>  27
<211>  26
<212>  PRT
<213>  Homo sapiens

<400>  27

Asp Ile Gln Met Thr Gln Ser Pro Ser Ser Leu Ser Ala Ser Val Gly
1               5                   10                  15

Asp Arg Val Thr Ile Thr Cys Ser Ala Ser
            20                  25
```

```
<210>  28
<211>  6
```

<212> PRT
<213> Homo sapiens

<400> 28

Gln Asp Ile Ser Asn Tyr
1 5

<210> 29
<211> 17
<212> PRT
<213> Homo sapiens

<400> 29

Leu Asn Trp Tyr Gln Gln Lys Pro Gly Lys Ala Pro Lys Leu Leu Ile
1 5 10 15

Tyr

<210> 30
<211> 4
<212> PRT
<213> Homo sapiens

<220>
<221> MISC_FEATURE
<222> (4)..(4)
<223> Xaa is not existed, just for finishing the sequence listing

<400> 30

Tyr Thr Ser Xaa
1

<210> 31
<211> 36
<212> PRT
<213> Homo sapiens

<400> 31

Asn Leu His Ser Gly Val Pro Ser Arg Phe Ser Gly Ser Gly Ser Gly
1 5 10 15

Thr Asp Phe Thr Leu Thr Ile Ser Ser Leu Gln Pro Glu Asp Phe Ala
20 25 30

Thr Tyr Tyr Cys
35

<210> 32
<211> 10
<212> PRT

<213> Homo sapiens

<400> 32

Gln Gln Ala Leu Val Val Thr Pro Phe Thr
1               5                   10

<210> 33
<211> 11
<212> PRT
<213> Homo sapiens

<400> 33

Phe Gly Gln Gly Thr Lys Leu Glu Ile Lys Arg
1               5                   10

<210> 34
<211> 25
<212> PRT
<213> Homo sapiens

<400> 34

Gln Val Gln Leu Val Gln Ser Gly Ala Glu Val Lys Lys Pro Gly Ser
1               5                   10                  15

Ser Val Lys Val Ser Cys Lys Ala Ser
            20                  25

<210> 35
<211> 8
<212> PRT
<213> Homo sapiens

<400> 35

Gly Gly Thr Phe Ser Asn Tyr Trp
1               5

<210> 36
<211> 17
<212> PRT
<213> Homo sapiens

<400> 36

Met His Trp Val Arg Gln Ala Pro Gly Gln Gly Leu Glu Trp Met Gly
1               5                   10                  15

Ala

<210> 37
<211> 8
<212> PRT

<213> Homo sapiens

<400> 37

Thr Tyr Arg Gly His Ser Asp Thr
1 5

<210> 38
<211> 38
<212> PRT
<213> Homo sapiens

<400> 38

Tyr Tyr Asn Gln Lys Phe Lys Gly Arg Val Thr Ile Thr Ala Asp Lys
1 5 10 15

Ser Thr Ser Thr Ala Tyr Met Glu Leu Ser Ser Leu Arg Ser Glu Asp
20 25 30

Thr Ala Val Tyr Tyr Cys
35

<210> 39
<211> 8
<212> PRT
<213> Homo sapiens

<400> 39

Ala Arg Phe Gly Met Leu Asp Asn
1 5

<210> 40
<211> 11
<212> PRT
<213> Homo sapiens

<400> 40

Trp Gly Gln Gly Thr Leu Val Thr Val Ser Ser
1 5 10

<210> 41
<211> 26
<212> PRT
<213> Homo sapiens

<400> 41

Asp Ile Gln Met Thr Gln Ser Pro Ser Ser Leu Ser Ala Ser Val Gly
1 5 10 15

Asp Arg Val Thr Ile Thr Cys Ser Ala Ser
20 25

<210> 42
<211> 6
<212> PRT
<213> Homo sapiens

<400> 42

Gln Asp Ile Ser Asn Tyr
1               5

<210> 43
<211> 17
<212> PRT
<213> Homo sapiens

<400> 43

Leu Asn Trp Tyr Gln Gln Lys Pro Gly Lys Ala Pro Lys Leu Leu Ile
1               5                   10                  15

Tyr

<210> 44
<211> 4
<212> PRT
<213> Homo sapiens

<220>
<221> MISC_FEATURE
<222> (4)..(4)
<223> Xaa is not existed, just for finishing the sequence listing

<400> 44

Tyr Thr Ser Xaa
1

<210> 45
<211> 36
<212> PRT
<213> Homo sapiens

<400> 45

Asn Leu His Ser Gly Val Pro Ser Arg Phe Ser Gly Ser Gly Ser Gly
1               5                   10                  15

Thr Asp Phe Thr Leu Thr Ile Ser Ser Leu Gln Pro Glu Asp Phe Ala
            20                  25                  30

Thr Tyr Tyr Cys
        35

<210> 46
<211> 10
<212> PRT
<213> Homo sapiens

<400> 46

Gln Gln Arg Leu Thr Pro Ser Pro Phe Thr
1 5 10

<210> 47
<211> 11
<212> PRT
<213> Homo sapiens

<400> 47

Phe Gly Gln Gly Thr Lys Leu Glu Ile Lys Arg
1 5 10

<210> 48
<211> 25
<212> PRT
<213> Homo sapiens

<400> 48

Gln Val Gln Leu Val Gln Ser Gly Ala Glu Val Lys Lys Pro Gly Ser
1 5 10 15

Ser Val Lys Val Ser Cys Lys Ala Ser
20 25

<210> 49
<211> 8
<212> PRT
<213> Homo sapiens

<400> 49

Gly Gly Thr Phe Ser Asn Tyr Trp
1 5

<210> 50
<211> 17
<212> PRT
<213> Homo sapiens

<400> 50

Met His Trp Val Arg Gln Ala Pro Gly Gln Gly Leu Glu Trp Met Gly
1 5 10 15

Ala

<210> 51
<211> 8
<212> PRT
<213> Homo sapiens

<400> 51

Thr Tyr Arg Gly His Ser Asp Thr
1 5

<210> 52
<211> 38
<212> PRT
<213> Homo sapiens

<400> 52

Tyr Tyr Asn Gln Lys Phe Lys Gly Arg Val Thr Ile Thr Ala Asp Lys
1 5 10 15

Ser Thr Ser Thr Ala Tyr Met Glu Leu Ser Ser Leu Arg Ser Glu Asp
20 25 30

Thr Ala Val Tyr Tyr Cys
35

<210> 53
<211> 9
<212> PRT
<213> Homo sapiens

<400> 53

Ala Arg Asn Thr Ala Leu Leu Asp Asn
1 5

<210> 54
<211> 11
<212> PRT
<213> Homo sapiens

<400> 54

Trp Gly Gln Gly Thr Leu Val Thr Val Ser Ser
1 5 10

<210> 55
<211> 60
<212> DNA
<213> Artificial Sequence

<220>
<223> Synthesized

<400> 55
gtgtattact gcgccarggs tkctrtttwc racgsttwcg amrttctgga caactggggc 60

<210> 56
<211> 48
<212> DNA
<213> Artificial Sequence

<220>
<223> Synthesized

<400> 56
tactactgcc agcagtwcar gargmtcsca tkgactttcg gccagggc 48

<210> 57
<211> 52
<212> DNA
<213> Artificial Sequence

<220>
<223> Synthesized

<220>
<221> misc_feature
<222> (24)..(25)
<223> n is a, c, g, or t

<220>
<221> misc_feature
<222> (27)..(28)
<223> n is a, c, g, or t

<400> 57
ccgctgtgta ttactgcgcc argnnknnkg stwtkctgga caactggggc ca 52

<210> 58
<211> 58
<212> DNA
<213> Artificial Sequence

<220>
<223> Synthesized

<220>
<221> misc_feature
<222> (24)..(25)
<223> n is a, c, g, or t

<220>
<221> misc_feature
<222> (27)..(28)
<223> n is a, c, g, or t

<220>
<221> misc_feature
<222> (30)..(31)
<223> n is a, c, g, or t

<220>
<221> misc_feature
<222> (33)..(34)

<223> n is a, c, g, or t

<400> 58
ccgctgtgta ttactgcgcc argnnknnkn nknnkgstwt kctggacaac tggggcca 58

<210> 59
<211> 73
<212> DNA
<213> Artificial Sequence

<220>
<223> Synthesized

<220>
<221> misc_feature
<222> (24)..(25)
<223> n is a, c, g, or t

<220>
<221> misc_feature
<222> (27)..(28)
<223> n is a, c, g, or t

<220>
<221> misc_feature
<222> (30)..(31)
<223> n is a, c, g, or t

<220>
<221> misc_feature
<222> (33)..(34)
<223> n is a, c, g, or t

<220>
<221> misc_feature
<222> (36)..(37)
<223> n is a, c, g, or t

<220>
<221> misc_feature
<222> (39)..(40)
<223> n is a, c, g, or t

<220>
<221> misc_feature
<222> (42)..(43)
<223> n is a, c, g, or t

<220>
<221> misc_feature
<222> (45)..(46)
<223> n is a, c, g, or t

<220>
<221> misc_feature
<222> (48)..(49)
<223> n is a, c, g, or t

<400> 59
ccgctgtgta ttactgcgcc argnnknnkn nknnknnknn knnknnknnk gstwtkctgg 60

acaactgggg cca 73

<210> 60
<211> 79
<212> DNA
<213> Artificial Sequence

<220>
<223> Synthesized

<220>
<221> misc_feature
<222> (24)..(25)
<223> n is a, c, g, or t

<220>
<221> misc_feature
<222> (27)..(28)
<223> n is a, c, g, or t

<220>
<221> misc_feature
<222> (30)..(31)
<223> n is a, c, g, or t

<220>
<221> misc_feature
<222> (33)..(34)
<223> n is a, c, g, or t

<220>
<221> misc_feature
<222> (36)..(37)
<223> n is a, c, g, or t

<220>
<221> misc_feature
<222> (39)..(40)
<223> n is a, c, g, or t

<220>
<221> misc_feature
<222> (42)..(43)
<223> n is a, c, g, or t

<220>
<221> misc_feature
<222> (45)..(46)
<223> n is a, c, g, or t

<220>
<221> misc_feature
<222> (48)..(49)
<223> n is a, c, g, or t

<220>
<221> misc_feature
<222> (51)..(52)
<223> n is a, c, g, or t

<220>

<221> misc_feature
<222> (54)..(55)
<223> n is a, c, g, or t

<400> 60
ccgctgtgta ttactgcgcc argnnknnkn nknnknnknn knnknnknnk nnknnkgstw 60

tkctggacaa ctggggcca 79

<210> 61
<211> 55
<212> DNA
<213> Artificial Sequence

<220>
<223> Synthesized

<220>
<221> misc_feature
<222> (21)..(22)
<223> n is a, c, g, or t

<220>
<221> misc_feature
<222> (24)..(25)
<223> n is a, c, g, or t

<220>
<221> misc_feature
<222> (27)..(28)
<223> n is a, c, g, or t

<220>
<221> misc_feature
<222> (30)..(31)
<223> n is a, c, g, or t

<400> 61
ccacctacta ctgccagcag nnknnknnkn nkcygwtcac tttcggccag ggcac 55

<210> 62
<211> 58
<212> DNA
<213> Artificial Sequence

<220>
<223> Synthesized

<220>
<221> misc_feature
<222> (21)..(22)
<223> n is a, c, g, or t

<220>
<221> misc_feature
<222> (24)..(25)
<223> n is a, c, g, or t

<220>

<221> misc_feature
<222> (27)..(28)
<223> n is a, c, g, or t

<220>
<221> misc_feature
<222> (30)..(31)
<223> n is a, c, g, or t

<220>
<221> misc_feature
<222> (33)..(34)
<223> n is a, c, g, or t

<400> 62
ccacctacta ctgccagcag nnknnknnkn nknnkcygwt cactttcggc cagggcac 58

<210> 63
<211> 70
<212> DNA
<213> Artificial Sequence

<220>
<223> Synthesized

<220>
<221> misc_feature
<222> (21)..(22)
<223> n is a, c, g, or t

<220>
<221> misc_feature
<222> (24)..(25)
<223> n is a, c, g, or t

<220>
<221> misc_feature
<222> (27)..(28)
<223> n is a, c, g, or t

<220>
<221> misc_feature
<222> (30)..(31)
<223> n is a, c, g, or t

<220>
<221> misc_feature
<222> (33)..(34)
<223> n is a, c, g, or t

<220>
<221> misc_feature
<222> (36)..(37)
<223> n is a, c, g, or t

<220>
<221> misc_feature
<222> (39)..(40)
<223> n is a, c, g, or t

<220>

<221> misc_feature
<222> (42)..(43)
<223> n is a, c, g, or t

<220>
<221> misc_feature
<222> (45)..(46)
<223> n is a, c, g, or t

<400> 63
ccacctacta ctgccagcag nnknnknnkn nknnknnknn knnknnkcyg wtcactttcg 60

gccagggcac 70

<210> 64
<211> 27
<212> DNA
<213> Artificial Sequence

<220>
<223> Synthesized

<400> 64
ctaccgtggc ccaggacatc cagatga 27

<210> 65
<211> 27
<212> DNA
<213> Artificial Sequence

<220>
<223> Synthesized

<400> 65
tggtgctggc cgggctggac acggtca 27

## Description

## Technical Field

The disclosure relates to the technical field of antibodies, and in particular to an anti-BCMA single-chain antibody scFv and a preparation method and application thereof.

## Background

In the past few years, the adoptive T cell immunotherapy has become one of the most promising strategies in cancer immunotherapy. CAR-T therapy has attracted a great attention of researchers since the birth thereof, especially in the treatment of patients with advanced leukemia in 2011, has achieved a great success. After remission of about 80% of lymphomas is achieved in 2012, it is rapidly developed. The current CAR-T therapy is mainly based on the second-generation CAR. The treatment mode is to isolate T cells from the patient and use engineered methods to equip the T cells with genetically modified chimeric antigen receptor (CAR). The CAR is a composite membrane receptor molecule, includes extracellular and intracellular two functional parts, and has functions of specifically positioning to a target molecule and activating the T cells. The extracellular part of the CAR is a recombinant receptor (scFv) formed by antibody single-chain variable fragments. It is mediated by the scFv, and specifically targeted to the target molecule in a mode of antigen-antibody specific binding, the T cells are activated, and thereby a tumor-killing effect is achieved.

After the successful treatment of blood diseases, people began to turn their attention to the field of solid tumors, including multiple myeloma, breast cancer, prostate cancer, ovarian cancer, glioma, colorectal cancer, liver metastasis, mesothelioma, kidney cancer, sarcoma, nerve cell carcinoma and pleural mesothelioma, etc. The year of 2017 was called the first year of cell therapy, French biotechnology company Cellectis announced in March that a universal CAR-T therapy UCART123 researched and developed by itself had been approved by the United States FDA for the phase-I clinical trial of an Investigative New Drug (IND), which was the first CAR-T clinical trial approved by the FDA in the world for an allogeneic method targeting CD123; and in August of the same year, the United States FDA approved the CD19-targeted CAR-T product Kymriah of Novartis; in October, Yescarta of Kite, a product used for the treatment of adult patients with specific-type large B-cell tumors, was launched, these achievements have greatly promoted the researchers' enthusiasm for the development of the cellular immunotherapy. As the latest technology of immunotherapy, CAR-T has a wide range of applications and broad prospects. The market value of CAR-T in China is expected to exceed 100 billion.

B Cell Maturation Antigen (BCMA, CD269) is a member of the tumor necrosis factor receptor (TNFR) superfamily and is a non-glycosylated type-III integral membrane protein composed of 185 amino acids. The BCMA binds the B cell activating factor (BAFF, also known as BLys, THANK, TALL-1) and a proliferation-inducing ligand (APRIL), and plays a vital role in processes of managing maturation of B cells and differentiating the B cells into plasma cells. It is indicated from researches that overexpression of the BCMA may cause generation of a protein kinase B, MAPK and NF-κB signals, thereby proliferation and survival of myeloma cells are enhanced. The BCMA is first found on the surface of mature B lymphocytes, and it is almost not expressed in other tissue cells, while it is highly expressed in malignant-proliferated B lymphocytes (such as myeloma cells and leukemia cells) and mediates a downstream signaling pathway. It has a critical effect on cell survival, proliferation, metastasis and drug resistance, these characteristics make it become a new drug target for diagnosis and immunotherapy of Multiple Myeloma (MM).

With the increasing incidence of MM and other B-cell-related diseases, a specific therapy for the B cells is expected to be developed clinically in a short period of time. In recent years, new tumor immunotherapy methods for the BCMA have become mature day by day, and mainly include a Chimeric Antigen Receptor T-Cell Immunotherapy (CAR-T), a bispecific antibody (BsAb) and an antibody-drug coupling (ADC). At an annual meeting of the American Association for Cancer Research (AACR) in April 2017, the BCMA becomes a star target, domestic and foreign companies rush to develop a BCMA-targeted CAR-T therapy. At present, 4 types of drugs have entered a clinical phase-I, and mainly include KITE-585, bb2121, LCAR-B38, etc. A domestic clinical application of the LCAR-B38 was submitted by Nanjing Legend Biotech Company in December 2017. It was the first domestic BCMA-CART to submit the clinical application. Data of a clinical trial involving 35 patients with relapsed or drug-resistant multiple myeloma showed that an objective remission rate of this therapy reached 100%, and 14 of the 19 patients reached the diagnostic criteria for a complete response during the early 4-month median follow-up period. At present, the research of the CAR-T therapy for the MM targeting to the BCMA is in an emerging stage. However, according to the results of pre-clinical and clinical phase-I trials, targeted drugs for the BCMA may be effectively targeted to tumor cells, killing the tumor cells through a cytotoxic effect of immune cells or small molecules, with significant drug effects and controllable adverse reactions, and it is a new target for the tumor targeted therapy with a great development potential.

With the spread of the multiple myeloma and other B cell-related diseases, the medical community urgently needs to develop a specific therapy for the B cells. However, technologies for the development of BCMA antibody drugs in the prior art are still focused on the use of a mouse-derived traditional antibody technology, and the traditional antibodies, whether large-scale expression or humanization, is more difficult, time-consuming, costly and low acquisition rate of effective antibodies, which severely limits the development of effective targeted drugs.

## Summary

The disclosure provides an anti-BCMA single-chain antibody scFv and a preparation method and application thereof.

According to a first aspect, an embodiment provides an anti-BCMA single-chain antibody scFv, including a light-chain variable region (VL) and a heavy-chain variable region (VH),

the above light-chain variable region (VL) includes a framework region (FR) and a complementarity determining region (CDR), herein the complementarity determining region (CDR) includes a CDR1, herein a sequence thereof is QDISNY (SEQ ID NO:14), a CDR2, herein a sequence thereof is YTS (SEQ ID NO:16), and a CDR3, herein a sequence thereof is QQYRKLAWT (SEQ ID NO:18); and the above heavy-chain variable region (VH) includes a framework region (FR) and a complementarity determining region (CDR), herein the complementarity determining region (CDR) includes a CDR1, herein a sequence thereof is GGTFSNYW (SEQ ID NO:21), a CDR2, herein a sequence thereof is TYRGHSDT (SEQ ID NO:23), and a CDR3, herein a sequence thereof is ARGAIYNGYDVLDN (SEQ ID NO:25); or

the above light-chain variable region (VL) includes a framework region (FR) and a complementarity determining region (CDR), herein the complementarity determining region (CDR) includes a CDR1, herein a sequence thereof is QDISNY (SEQ ID NO:28), a CDR2, herein a sequence thereof is YTS (SEQ ID NO:30), and a CDR3, herein a sequence thereof is QQALVVTPFT (SEQ ID NO:32); and the above heavy-chain variable region (VH) includes a framework region (FR) and a complementarity determining region (CDR), herein the complementarity determining region (CDR) includes a CDR1, herein a sequence thereof is GGTFSNYW (SEQ ID NO:35), a CDR2, herein a sequence thereof is TYRGHSDT (SEQ ID NO:37), and a CDR3, herein a sequence thereof is ARFGMLDN (SEQ ID NO:39); or

the above light-chain variable region (VL) includes a framework region (FR) and a complementarity determining region (CDR), herein the complementarity determining region (CDR) includes a CDR1, herein a sequence thereof is QDISNY (SEQ ID NO:42), a CDR2, herein a sequence thereof is YTS (SEQ ID NO:44), and a CDR3, herein a sequence thereof is QQRLTPSPFT(SEQ ID NO:46); and the above heavy-chain variable region (VH) includes a framework region (FR) and a complementarity determining region (CDR), herein the complementarity determining region (CDR) includes a CDR1, herein a sequence thereof is GGTFSNYW (SEQ ID NO:49), a CDR2, herein a sequence thereof is TYRGHSDT (SEQ ID NO:51), and a CDR3, herein a sequence thereof is ARNTALLDN (SEQ ID NO:53).

Preferably, the above light-chain variable region (VL) is:

DIQMTQSPSSLSASVGDRVTITCSASQDISNYLNWYQQKPGKAPKLLIYYTSNLHSGVPSRFS GSGSGTDFTLTISSLQPEDFATYYCQQYRKLAWTFGQGTKLEIKR (SEQ ID NO:4);

the above heavy-chain variable region (VH) is:

QVQLVQSGAEVKKPGSSVKVSCKASGGTFSNYWMHWVRQAPGQGLEWMGATYRGHSDTY YNQKFKGRVTITADKSTSTAYMELSSLRSEDTAVYYCARGSIFNGYDVLDNWGQGTLVTVSS (SEQ ID NO:3);

or

the above light-chain variable region (VL) is:

DIQMTQSPSSLSASVGDRVTITCSASQDISNYLNWYQQKPGKAPKLLIYYTSNLHSGVPSRFS GSGSGTDFTLTISSLQPEDFATYYCQQALVVTPFTFGQGTKLEIKR (SEQ ID NO:8);

the above heavy-chain variable region (VH) is:

QVQLVQSGAEVKKPGSSVKVSCKASGGTFSNYWMHWVRQAPGQGLEWMGATYRGHSDTY YNQKFKGRVTITADKSTSTAYMELSSLRSEDTAVYYCARFGMLDNWGQGTLVTVSS (SEQ ID NO:7);

or

the above light-chain variable region (VL) is:

DIQMTQSPSSLSASVGDRVTITCSASQDISNYLNWYQQKPGKAPKLLIYYTSNLHSGVPSRFS GSGSGTDFTLTISSLQPEDFATYYCQQRLTPSPFTFGQGTKLEIK (SEQ ID NO:12);

the above heavy-chain variable region (VH) is:

QVQLVQSGAEVKKPGSSVKVSCKASGGTFSNYWMHWVRQAPGQGLEWMGATYRGHSDTY YNQKFKGRVTITADKSTSTAYMELSSLRSEDTAVYYCARNTALLDNWGQGTLVTVSS (SEQ ID NO:11).

Preferably, a sequence of the above single-chain antibody scFv is:

DIQMTQSPSSLSASVGDRVTITCSASQDISNYLNWYQQKPGKAPKLLIYYTSNLHSGVPSRFS GSGSGTDFTLTISSLQPEDFATYYCQQYRKLAWTFGQGTKLEIKRGGGGSGGGGSGGGGSGGG GSQVQLVQSGAEVKKPGSSVKVSCKASGGTFSNYWMHWVRQAPGQGLEWMGATYRGHSDTY YNQKFKGRVTITADKSTSTAYMELSSLRSEDTAVYYCARGSIFNGYDVLDNWGQGTLVTVSS (SEQ ID NO:1);

or

DIQMTQSPSSLSASVGDRVTITCSASQDISNYLNWYQQKPGKAPKLLIYYTSNLHSGVPSRFS GSGSGTDFTLTISSLQPEDFATYYCQQALVVTPFTFGQGTKLEIKRGGGGSGGGGSGGGGSGGG GSQVQLVQSGAEVKKPGSSVKVSCKASGGTFSNYWMHWVRQAPGQGLEWMGATYRGHSDTY YNQKFKGRVTITADKSTSTAYMELSSLRSEDTAVYYCARFGMLDNWGQGTLVTVSS (SEQ ID NO:5);

or

DIQMTQSPSSLSASVGDRVTITCSASQDISNYLNWYQQKPGKAPKLLIYYTSNLHSGVPSRFS GSGSGTDFTLTISSLQPEDFATYYCQQRLTPSPFTFGQGTKLEIKRGGGGSGGGGSGGGGSGGG GSQVQLVQSGAEVKKPGSSVKVSCKASGGTFSNYWMHWVRQAPGQGLEWMGATYRGHSDTY YNQKFKGRVTITADKSTSTAYMELSSLRSEDTAVYYCARNTALLDNWGQGTLVTVSS (SEQ ID NO:9).

According to a second aspect, an embodiment provides a polynucleotide sequence for encoding the anti-BCMA single-chain antibody scFv in the first aspect, including a nucleotide sequence for encoding complementarity determining region sequences SEQ ID NO: 14, SEQ ID NO: 16 and SEQ ID NO: 18 of the above light-chain variable region (VL); and a nucleotide sequence for encoding complementarity determining region sequences SEQ ID NO: 21, SEQ ID NO: 23, and SEQ ID NO: 25 of the above heavy-chain variable region (VH); or

the above polynucleotide sequence includes a nucleotide sequence for encoding complementarity determining region sequences SEQ ID NO: 28, SEQ ID NO: 30 and SEQ ID NO: 32 of the above light-chain variable region (VL); and a nucleotide sequence for encoding complementarity determining region sequences SEQ ID NO: 35, SEQ ID NO: 37, and SEQ ID NO: 39 of the above heavy-chain variable region (VH); or

the above polynucleotide sequence includes a nucleotide sequence for encoding complementarity determining region sequences SEQ ID NO: 42, SEQ ID NO: 44 and SEQ ID NO: 46 of the above light-chain variable region (VL); and a nucleotide sequence for encoding complementarity determining region sequences SEQ ID NO: 49, SEQ ID NO: 51, and SEQ ID NO: 53 of the above heavy-chain variable region (VH).

Preferably, the above polynucleotide sequence includes a nucleotide sequence for encoding a sequence SEQ ID NO:4 of the above light-chain variable region (VL); and a nucleotide sequence for encoding a sequence SEQ ID NO:3 of the above heavy-chain variable region (VH); or

the above polynucleotide sequence includes a nucleotide sequence for encoding a sequence SEQ ID NO:8 of the above light-chain variable region (VL); and a nucleotide sequence for encoding a sequence SEQ ID NO:7 of the above heavy-chain variable region (VH); or

the above polynucleotide sequence includes a nucleotide sequence for encoding a sequence SEQ ID NO:12 of the above light-chain variable region (VL); and a nucleotide sequence for encoding a sequence SEQ ID NO:11 of the above heavy-chain variable region (VH).

Preferably, the above polynucleotide sequence is:

GACATCCAGATGACCCAGAGCCCTAGCTCACTGAGCGCCAGCGTGGGCGACAGGGTGA
CCATTACCTGCTCCGCCAGCCAGGACATCAGCAACTACCTGAACTGGTACCAGCAGAAGCCC
GGCAAGGCCCCCAAGCTGCTGATCTACTACACCTCCAACCTGCACTCCGGCGTGCCCAGCAG
GTTCAGCGGAAGCGGCAGCGGCACCGATTTCACCCTGACCATCTCCAGCCTGCAGCCCGAG
GACTTCGCCACCTACTACTGCCAGCAGTACAGGAAGCTCGCATGGACTTTCGGCCAGGGCAC
CAAACTGGAGATCAAGCGTGGTGGAGGAGGTAGCGGAGGAGGCGGGAGCGGTGGAGGTGG
CTCTGGAGGTGGCGGAAGCCAGGTGCAGCTGGTCCAGAGCGGCGCCGAAGTGAAGAAGCC
CGGCAGCTCCGTGAAAGTGAGCTGCAAGGCCAGCGGCGGCACCTTCAGCAACTACTGGATG
CACTGGGTGAGGCAGGCCCCCGGACAGGGCCTGGAGTGGATGGGCGCCACCTACAGGGGC
CACAGCGACACCTACTACAACCAGAAGTTCAAGGGCCGGGTGACCATCACCGCCGACAAGAG
CACCAGCACCGCCTACATGGAACTGAGCAGCCTCAGGAGCGAGGACACCGCTGTGTATTACT
GCGCCAGGGGTTCTATTTTCAACGGTTACGACGTTCTGGACAACTGGGGCCAGGGCACACTA
GTGACCGTGTCCAGC (SEQ ID NO:2);

or

GACATCCAGATGACCCAGAGCCCTAGCTCACTGAGCGCCAGCGTGGGCGACAGGGTGA CCATTACCTGCTCCGCCAGCCAGGACATCAGCAACTACCTGAACTGGTACCAGCAGAAGCCC GGCAAGGCCCCCAAGCTGCTGATCTACTACACCTCCAACCTGCACTCCGGCGTGCCCAGCAG GTTCAGCGGAAGCGGCAGCGGCACCGATTTCACCCTGACCATCTCCAGCCTGCAGCCCGAG GACTTCGCCACCTACTACTGCCAGCAGGCTCTTGTGGTGACGCCGTTCACTTTCGGCCAGGG CACCAAACTGGAGATCAAGCGTGGTGGAGGAGGTAGCGGAGGAGGCGGGAGCGGTGGAGG TGGCTCTGGAGGTGGCGGAAGCCAGGTGCAGCTGGTCCAGAGCGGCGCCGAAGTGAAGAA GCCCGGCAGCTCCGTGAAAGTGAGCTGCAAGGCCAGCGGCGGCACCTTCAGCAACTACTGG ATGCACTGGGTGAGGCAGGCCCCCGGACAGGGCCTGGAGTGGATGGGCGCCACCTACAGG GGCCACAGCGACACCTACTACAACCAGAAGTTCAAGGGCCGGGTGACCATCACCGCCGACAA GAGCACCAGCACCGCCTACATGGAACTGAGCAGCCTCAGGAGCGAGGACACCGCTGTGTATT ACTGCGCCAGGTTTTAGGGTATGCTGGACAACTGGGGCCAGGGCACACTAGTGACCGTGTCC AGC (SEQ ID NO:6);

or

GACATCCAGATGACCCAGAGCCCTAGCTCACTGAGCGCCAGCGTGGGCGACAGGGTGA CCATTACCTGCTCCGCCAGCCAGGACATCAGCAACTACCTGAACTGGTACCAGCAGAAGCCC GGCAAGGCCCCCAAGCTGCTGATCTACTACACCTCCAACCTGCACTCCGGCGTGCCCAGCAG GTTCAGCGGAAGCGGCAGCGGCACCGATTTCACCCTGACCATCTCCAGCCTGCAGCCCGAG GACTTCGCCACCTACTACTGCCAGCAGCGTTTGACGCCGTCTCCGTTCACTTTCGGCCAGGG CACCAAACTGGAGATCAAGCGTGGTGGAGGAGGTAGCGGAGGAGGCGGGAGCGGTGGAGG TGGCTCTGGAGGTGGCGGAAGCCAGGTGCAGCTGGTCCAGAGCGGCGCCGAAGTGAAGAA GCCCGGCAGCTCCGTGAAAGTGAGCTGCAAGGCCAGCGGCGGCACCTTCAGCAACTACTGG ATGCACTGGGTGAGGCAGGCCCCCGGACAGGGCCTGGAGTGGATGGGCGCCACCTACAGG GGCCACAGCGACACCTACTACAACCAGAAGTTCAAGGGCCGGGTGACCATCACCGCCGACAA GAGCACCAGCACCGCCTACATGGAACTGAGCAGCCTCAGGAGCGAGGACACCGCTGTGTATT ACTGCGCCAGGAATACGGCTTTGCTGGACAACTGGGGCCAGGGCACACTAGTGACCGTGTCC AGC (SEQ ID NO:10).

According to a third aspect, an embodiment provides an expression vector, containing the polynucleotide sequence in the second aspect.

According to a fourth aspect, an embodiment provides a host cell, containing the expression vector in the third aspect, and an anti-BCMA single-chain antibody scFv may be expressed.

According to a fifth aspect, an embodiment provides a pharmaceutical composition, containing the anti-BCMA single-chain antibody scFv in the first aspect, and a pharmaceutically acceptable carrier, a diluent or a excipient.

According to a sixth aspect, an embodiment provides a method for preparing the anti-BCMA single-chain antibody scFv in the first aspect, including: transforming an expression vector containing the polynucleotide sequence in the second aspect to an expression host cell, culturing, and performing mass expression and purification of the above anti-BCMA single-chain antibody scFv.

Preferably, the above expression vector is a pComb3XSS vector, and the above host cells are Escherichia coli XL1-Blue and a TG1 strain.

According to a seventh aspect, an embodiment provides an application of the anti-BCMA single-chain antibody scFv in the first aspect in preparing a BCMA-targeted drug, or an application in immunologically detecting BCMA for a non-disease diagnosis and treatment purpose.

A BCMA-targeted phage display library is artificially synthesized in the disclosure in combination with site-directed mutagenesis and random mutagenesis technologies, and 3 new-type BCMA antigen-targeted scFv strains are obtained by combining a phage display technology, which may be served as a BCMA-targeted antibody for further research and development.

## Brief Description of the Drawings

Fig. 1 is an electrophoresis detection result diagram of a secondary library and a random library of site-directed mutagenesis in Embodiment 1 of the disclosure.

Fig. 2 is a recombinant efficiency result diagram of a scFv fragment in a phage display library constructed by cloning PCR verification in Embodiment 1 of the disclosure, herein clones 1-36 are clones of the secondary library, and clones 37-72 are clones of the random library.

Fig. 3 is an affinity result diagram of phage library targeting BCMA after 4 rounds of affinity biopanning detected by ELISA in Embodiment 2 of the disclosure.

Fig. 4 is a BCMA-targeted scFv monoclonal PCR screening result diagram in Embodiment 2 of the disclosure, herein clones 1-36 are clones screened from the random library, clones 37-56 are clones screened from the secondary library, and the arrow represents a clone with a mutated sequence.

Fig. 5 is an affinity result diagram of a BCMA-targeted scFv candidate strain verified by the ELISA in Embodiment 2 of the disclosure.

## Detailed Description of the Embodiments

The disclosure is further described in detail below through specific implementation modes in combination with drawings. In the following implementation modes, many detailed descriptions are used to enable the disclosure to be better understood. However, it may be easily realized by those skilled in the art that some of features may be omitted under different circumstances, or may be replaced by other elements, materials, and methods.

In addition, features, operations, or characteristics described in the description may be combined in any suitable modes to form the various implementation modes. At the same time, each operation or action described in the methods may also be sequentially exchanged or adjusted in a mode which is apparent to those skilled in the art. Therefore, various sequences in the description and the drawings are only used for clearly describing a certain embodiment, and are not meant to be a necessary sequence, unless it is otherwise specified that a certain sequence must be followed.

In the present disclosure, based on a humanized scFv sequence of anti-BCMA, site-directed mutagenesis and random mutagenesis are performed on CDR3 regions of heavy-chain and light-chain thereof, and a phage library of anti-BCMA scFv is artificially synthesized; then combined with a phage display technology, multiple rounds of affinity panning and screening are performed on the BCMA antigen, finally sequence analysis and affinity detection are performed on the antibody monoclonal strains obtained from screening, and new anti-BCMA scFv strains have been obtained and can be used as an antibody of a BCMA-targeted drug for the development of downstream immunotherapeutic drugs .

In the present disclosure, the humanized scFv sequence is used as a backbone, and only the CDR3 regions thereof are artificially modified, which can greatly reduce the difficulty of humanization of the antibody in the later stage, and meanwhile reduce the effect of the modification on the affinity of the antibody targeted to the antigen; at the same time, by combining the use of the phage display technology, antibody affinity information is obtained relatively quickly and intuitively and multiple new high-affinity scFv strains are obtained in a short time, which provides more ways for development of the BCMA-targeted immunotherapy, and has a value of continued development.

Based on the scFv sequence of BCMA, the CDR3 regions of the heavy-chain and light-chain thereof are mutated, and 3 new BCMA-targeted scFv antibody strains are obtained by combining with the phage display technology.

The amino acid and nucleotide sequence information of the 3 anti-BCMA scFv antibodies obtained above are as shown in Table 1:

Table 1: Nucleotide and amino acid sequence information of anti-BCMA positive clones scFv_20, scFv_43 and scFv_46

| Name/ Description | Sequence |
|---|---|
| scFv_20 | |
| scFv_20-aa scFv domain (domain) | DIQMTQSPSSLSASVGDRVTITCSASQDISNYLNWYQQKPGKAPKLLIYYTSNLHSGVPSRFSGSGSGTDFTLTISSLQPEDFATYYCQQYRKLAWTFGQGTKLEIKRGGGGSGGGGSGGGGSGGGGSQVQLVQSGAEVKKPGSSVKVSCKASGGTFSNYWMHWVRQAPGQGLEWMGATYRGHSDTYYNQKFKGRVTITADKSTSTAYMELSSLRSEDTAVYYCARGSIFNGYDVLDNWGQGTLVTVSS (SEQ ID NO:1) |
| scFv_20-nt scFv domain (domain) | GACATCCAGATGACCCAGAGCCCTAGCTCACTGAGCGCCAGCGTGGGCGACAGGGTGACCATTACCTGCTCCGCCAGCCAGGACATCAGCAACTACCTGAACTGGTACCAGCAGAAGCCCGGCAAGGCCCCCAAGCTGCTGATCTACTACACCTCCAACCTGCACTCCGGCGTGCCCAGCAGGTTCAGCGGAAGCGGCAGCGGCACCGATTTCACCCTGACCATCTCCAGCCTGCAGCCCGAGGACTTCGCCACCTACTACTGCCAGCAGTACAGGAAGCTCGCATGGACTTTCGGCCAGGGCACCAAACTGGAGATCAAGCGTGGTGGAGGAGGTAGCGGAGGAGGCGGGAGCGGTGGAGGTGGCTCTGGAGGTGGCGGAAGCCAGGTGCAGCTGGTCCAGAGCGGCGCCGAAGTGAAGAAGCCCGGCAGCTCCGTGAAAGTGAGCTGCAAGGCCAGCGGCGGCACCTTCAGCAACTACTGGATGCACTGGGTGAGGCAGGCCCCCGGACAGGGCCTGGAGTGGATGGGCGCCACCTACAGGGGCCACAGCGACACCTACTACAACCAGAAGTTCAAGGGCCGGGTGACCATCACCGCCGACAAGAGCACCAGCACCGCCTACATGGAACTGAGCAGCCTCAGGAGCGAGGACACCGCTGTGTATTACTGCGCCAGGGGTTCTATTTTCAACGGTTACGACGTTCTGGACAACTGGGGCCAGGGCACACTAGTGACCGTGTCCAGC (SEQ ID NO:2) |
| scFv_20-aaVH (heavy-chain variable region) | QVQLVQSGAEVKKPGSSVKVSCKASGGTFSNYWMHWVRQAPGQGLEWMGATYRGHSDTYYNQKFKGRVTITADKSTSTAYMELSSLRSEDTAVYYCARGSIFNGYDVLDNWGQGTLVTVSS (SEQ ID NO:3) |
| scFv_20-aa VL (light-chain variable region) | DIQMTQSPSSLSASVGDRVTITCSASQDISNYLNWYQQKPGKAPKLLIYYTSNLHSGVPSRFSGSGSGTDFTLTISSLQPEDFATYYCQQYRKLAWTFGQGTKLEIKR (SEQ ID NO:4) |

(continued)

| scFv_43 | |
|---|---|
| scFv_43-aa scFv domain (domain) | DIQMTQSPSSLSASVGDRVTITCSASQDISNYLNWYQQKPGKAPKLLIYYTSNLHS GVPSRFSGSGSGTDFTLTISSLQPEDFATYYCQQALVVTPFTFGQGTKLEIKRGGG GSGGGGSGGGGSGGGGSQVQLVQSGAEVKKPGSSVKVSCKASGGTFSNYWM HWVRQAPGQGLEWMGATYRGHSDTYYNQKFKGRVTITADKSTSTAYMELSSLRS EDTAVYYCARFGMLDNWGQGTLVTVSS (SEQ ID NO:5) |
| scFv_43-nt scFv domain (domain) | GACATCCAGATGACCCAGAGCCCTAGCTCACTGAGCGCCAGCGTGGGCGACA GGGTGACCATTACCTGCTCCGCCAGCCAGGACATCAGCAACTACCTGAACTG GTACCAGCAGAAGCCCGGCAAGGCCCCCAAGCTGCTGATCTACTACACCTCC AACCTGCACTCCGGCGTGCCCAGCAGGTTCAGCGGAAGCGGCAGCGGCACC GATTTCACCCTGACCATCTCCAGCCTGCAGCCCGAGGACTTCGCCACCTACTA CTGCCAGCAGGCTCTTGTGGTGACGCCGTTCACTTTCGGCCAGGGCACCAAA CTGGAGATCAAGCGTGGTGGAGGAGGTAGCGGAGGAGGCGGGAGCGGTGGA GGTGGCTCTGGAGGTGGCGGAAGCCAGGTGCAGCTGGTCCAGAGCGGCGCC GAAGTGAAGAAGCCCGGCAGCTCCGTGAAAGTGAGCTGCAAGGCCAGCGGC GGCACCTTCAGCAACTACTGGATGCACTGGGTGAGGCAGGCCCCCGGACAGG GCCTGGAGTGGATGGGCGCCACCTACAGGGGCCACAGCGACACCTACTACAA CCAGAAGTTCAAGGGCCGGGTGACCATCACCGCCGACAAGAGCACCAGCACC GCCTACATGGAACTGAGCAGCCTCAGGAGCGAGGACACCGCTGTGTATTACT GCGCCAGGTTTTAGGGTATGCTGGACAACTGGGGCCAGGGCACACTAGTGAC CGTGTCCAGC (SEQ ID NO:6) |
| scFv_43-aaVH (heavy-chain variable region) | QVQLVQSGAEVKKPGSSVKVSCKASGGTFSNYWMHWVRQAPGQGLEWMGATY RGHSDTYYNQKFKGRVTITADKSTSTAYMELSSLRSEDTAVYYCARFGMLDNWG QGTLVTVSS (SEQ ID NO:7) |
| scFv_43-aaVL (light-chain variable region) | DIQMTQSPSSLSASVGDRVTITCSASQDISNYLNWYQQKPGKAPKLLIYYTSNLHS GVPSRFSGSGSGTDFTLTISSLQPEDFATYYCQQALVVTPFTFGQGTKLEIKR (SEQ ID NO:8) |
| scFv_46 | |
| scFv_46-aa scFv domain (domain) | DIQMTQSPSSLSASVGDRVTITCSASQDISNYLNWYQQKPGKAPKLLIYYTSNLHS GVPSRFSGSGSGTDFTLTISSLQPEDFATYYCQQRLTPSPFTFGQGTKLEIKRGG GGSGGGGSGGGGSGGGGSQVQLVQSGAEVKKPGSSVKVSCKASGGTFSNYW MHWVRQAPGQGLEWMGATYRGHSDTYYNQKFKGRVTITADKSTSTAYMELSSL RSEDTAVYYCARNTALLDNWGQGTLVTVSS (SEQ ID NO:9) |

(continued)

| scFv_46 | |
|---|---|
| scFv_46-nt scFv domain (domain) | GACATCCAGATGACCCAGAGCCCTAGCTCACTGAGCGCCAGCGTGGGCGACA GGGTGACCATTACCTGCTCCGCCAGCCAGGACATCAGCAACTACCTGAACTG GTACCAGCAGAAGCCCGGCAAGGCCCCCAAGCTGCTGATCTACTACACCTCC AACCTGCACTCCGGCGTGCCCAGCAGGTTCAGCGGAAGCGGCAGCGGCACC GATTTCACCCTGACCATCTCCAGCCTGCAGCCCGAGGACTTCGCCACCTACTA CTGCCAGCAGCGTTTGACGCCGTCTCCGTTCACTTTCGGCCAGGGCACCAAA CTGGAGATCAAGCGTGGTGGAGGAGGTAGCGGAGGAGGCGGGAGCGGTGGA GGTGGCTCTGGAGGTGGCGGAAGCCAGGTGCAGCTGGTCCAGAGCGGCGCC GAAGTGAAGAAGCCCGGCAGCTCCGTGAAAGTGAGCTGCAAGGCCAGCGGC GGCACCTTCAGCAACTACTGGATGCACTGGGTGAGGCAGGCCCCCGGACAGG GCCTGGAGTGGATGGGCGCCACCTACAGGGGCCACAGCGACACCTACTACAA CCAGAAGTTCAAGGGCCGGGTGACCATCACCGCCGACAAGAGCACCAGCACC GCCTACATGGAACTGAGCAGCCTCAGGAGCGAGGACACCGCTGTGTATTACT GCGCCAGGAATACGGCTTTGCTGGACAACTGGGGCCAGGGCACACTAGTGAC CGTGTCCAGC (SEQ ID NO:10) |
| scFv_46-aa VH (heavy-chain variable region) | QVQLVQSGAEVKKPGSSVKVSCKASGGTFSNYWMHWVRQAPGQGLEWMGATY RGHSDTYYNQKFKGRVTITADKSTSTAYMELSSLRSEDTAVYYCARNTALLDNWG QGTLVTVSS (SEQ ID NO:11) |
| scFv_46-aa VL (light-chain variable region) | DIQMTQSPSSLSASVGDRVTITCSASQDISNYLNWYQQKPGKAPKLLIYYTSNLHS GVPSRFSGSGSGTDFTLTISSLQPEDFATYYCQQRLTPSPFTFGQGTKLEIK (SEQ ID NO:12) |

Amino acid sequences of the framework regions (FR) and complementarity determining regions (CDR) of the light-chains (L chain) and the heavy-chains (H chain) of the 3 scFv strains obtained above are as shown in Table 2:

Table 2: Amino acid sequence information of the framework regions (FR1-FR4) and complementarity determining regions (CDR1-CDR3) of the light-chains and heavy-chains of the anti-BCMA positive clones scFv_20, scFv_43 and scFv_23

| Clone strain | Chain | FR1 | CDR1 | FR2 | CDR2 | FR3 | CDR3 | FR4 |
|---|---|---|---|---|---|---|---|---|
| scFv_ 20 | Light-chain | DIQMTQ SPSSLSA SVGDRV TITCSAS (SEQ ID NO:13) | QDISNY (SEQ ID NO:14) | LNWYQQ KPGKAP KLLIY (SEQ ID NO:15) | YTS (SEQ ID NO:16) | NLHSGVPS RFSGSGSG TDFTLTISSL QPEDFATY YC (SEQ ID NO:17) | QQYRKL AWT (SEQ ID NO:18) | FGQGTKL EIKR (SEQ ID NO:19) |
| scFv_ 20 | Heavy-chain | QVQLVQ SGAEVK KPGSSV KVSCKA S (SEQ ID NO:20) | GGTFS NYW (SEQ ID NO:21) | MHWVR QAPGQG LEWMGA (SEQ ID NO:22) | TYRG HSDT (SEQ ID NO:23 ) | YYNQKFKG RVTITADKS TSTAYMELS SLRSEDTAV YYC (SEQ ID NO:24) | ARGAIYN GYDVLD N (SEQ ID NO:25) | WGQGTLV TVSS (SEQ ID NO:26) |
| scFv_ 43 | Light-chain | DIQMTQ SPSSLSA SVGDRV TITCSAS (SEQ ID NO:27) | QDISNY (SEQ ID NO:28) | LNWYQQ KPGKAP KLLIY (SEQ ID NO:29) | YTS (SEQ ID NO:30) | NLHSGVPS RFSGSGSG TDFTLTISSL QPEDFATY YC (SEQ ID NO:31) | QQALVV TPFT (SEQ ID NO:32) | FGQGTKL EIKR (SEQ ID NO:33) |

(continued)

| Clone strain | Chain | FR1 | CDR1 | FR2 | CDR2 | FR3 | CDR3 | FR4 |
|---|---|---|---|---|---|---|---|---|
| scFv_ 43 | Heavy-chain | QVQLVQ SGAEVK KPGSSV KVSCKA S (SEQ ID NO:34) | GGTFS NYW (SEQ ID NO:35) | MHWVR QAPGQG LEWMGA (SEQ ID NO:36) | TYRG HSDT (SEQ ID NO:37 ) | YYNQKFKG RVTITADKS TSTAYMELS SLRSEDTAV YYC (SEQ ID NO:38) | ARFGML DN (SEQ ID NO:39) | WGQGTLV TVSS (SEQ ID NO:40) |
| scFv_ 46 | Light-chain | DIQMTQ SPSSLSA SVGDRV TITCSAS (SEQ ID NO:41) | QDISNY (SEQ ID NO:42) | LNWYQQ KPGKAP KLLIY (SEQ ID NO:43) | YTS (SEQ ID NO:44) | NLHSGVPS RFSGSGSG TDFTLTISSL QPEDFATY YC (SEQ ID NO:45) | QQRLTP SPFT (SEQ ID NO:46) | FGQGTKL EIKR (SEQ ID NO:47) |
| scFv_ 46 | Heavy-chain | QVQLVQ SGAEVK KPGSSV KVSCKA S (SEQ ID NO:48) | GGTFS NYW (SEQ ID NO:49) | MHWVR QAPGQG LEWMGA (SEQ ID NO:50) | TYRG HSDT (SEQ ID NO:51 ) | YYNQKFKG RVTITADKS TSTAYMELS SLRSEDTAV YYC (SEQ ID NO:52) | ARNTALL DN (SEQ ID NO:53) | WGQGTLV TVSS (SEQ ID NO:54) |

The scFv antibody obtained in the disclosure may be efficiently targeted and bound to the BCMA antigen, and is applied to a purpose of BCMA-targeted diagnosis or treatment.

It should be to be noted that it is well-known that specific binding properties of the antibodies are determined by the complementarity determining regions. Therefore, a technical scheme of the disclosure provides an anti-BCMA single-chain antibody scFv, including a light-chain variable region (VL) and a heavy-chain variable region (VH), the light-chain variable region (VL) includes a framework region (FR) and a complementarity determining region (CDR), herein the complementarity determining region (CDR) includes a CDR1, herein a sequence thereof is QDISNY (SEQ ID NO:14), a CDR2, herein a sequence thereof is YTS (SEQ ID NO:16), and a CDR3, herein a sequence thereof is QQYRKLAWT (SEQ ID NO:18); and the heavy-chain variable region (VH) includes a framework region (FR) and a complementarity determining region (CDR), herein the complementarity determining region (CDR) includes a CDR1, herein a sequence thereof is GGTFSNYW (SEQ ID NO:21), a CDR2, herein a sequence thereof is TYRGHSDT (SEQ ID NO:23), and a CDR3, herein a sequence thereof is ARGAIYNGYDVLDN (SEQ ID NO:25); or the light-chain variable region (VL) includes a framework region (FR) and a complementarity determining region (CDR), herein the complementarity determining region (CDR) includes a CDR1, herein a sequence thereof is QDISNY (SEQ ID NO:28), a CDR2, herein a sequence thereof is YTS (SEQ ID NO:30), and a CDR3, herein a sequence thereof is QQALVVTPFT (SEQ ID NO:32); and the heavy-chain variable region (VH) includes a framework region (FR) and a complementarity determining region (CDR), herein the complementarity determining region (CDR) includes a CDR1, herein a sequence thereof is GGTFSNYW (SEQ ID NO:35), a CDR2, herein a sequence thereof is TYRGHSDT (SEQ ID NO:37), and a CDR3, herein a sequence thereof is ARFGMLDN (SEQ ID NO:39); or the light-chain variable region (VL) includes a framework region (FR) and a complementarity determining region (CDR), herein the complementarity determining region (CDR) includes a CDR1, herein a sequence thereof is QDISNY (SEQ ID NO:42), a CDR2, herein a sequence thereof is YTS (SEQ ID NO:44), and a CDR3, herein a sequence thereof is QQRLTPSPFT (SEQ ID NO:46); and the heavy-chain variable region (VH) includes a framework region (FR) and a complementarity determining region (CDR), herein the complementarity determining region (CDR) includes a CDR1, herein a sequence thereof is GGTFSNYW (SEQ ID NO:49), a CDR2, herein a sequence thereof is TYRGHSDT (SEQ ID NO:51), and a CDR3, herein a sequence thereof is ARNTALLDN (SEQ ID NO:53).

In a preferred technical scheme, the anti-BCMA single-chain antibody scFv includes a light-chain variable region (VL) and a heavy-chain variable region (VH), herein, a sequence of the light-chain variable region (VL) is SEQ ID NO:4, and a sequence of the heavy-chain variable region (VH) is SEQ ID NO:3; or a sequence of the light-chain variable region (VL) is SEQ ID NO:8, and a sequence of the heavy-chain variable region (VH) is SEQ ID NO:7; or a sequence of the light-chain variable region (VL) is SEQ ID NO:12, and a sequence of the heavy-chain variable region (VH) is SEQ ID NO:11.

In a more preferred technical scheme, a sequence of the anti-BCMA single-chain antibody scFv is SEQ ID NO:1; or SEQ ID NO:5; or SEQ ID NO:9.

In view of the degeneracy of an encoding gene, and at the same time, it is considered that the specific binding characteristics of the antibodies are determined by the complementarity determining regions. Therefore, a technical scheme of the disclosure provides a polynucleotide sequence for encoding the anti-BCMA single-chain antibody scFv of the disclosure, including a nucleotide sequence for encoding complementarity determining region sequences SEQ ID NO: 14, SEQ ID NO: 16 and SEQ ID NO: 18 of the light-chain variable region (VL); and a nucleotide sequence for encoding complementarity determining region sequences SEQ ID NO: 21, SEQ ID NO: 23, and SEQ ID NO: 25 of the heavy-chain variable region (VH); or the polynucleotide sequence includes a nucleotide sequence for encoding complementarity determining region sequences SEQ ID NO: 28, SEQ ID NO: 30 and SEQ ID NO: 32 of the light-chain variable region (VL); and a nucleotide sequence for encoding complementarity determining region sequences SEQ ID NO: 35, SEQ ID NO: 37, and SEQ ID NO: 39 of the heavy-chain variable region (VH); or the polynucleotide sequence includes a nucleotide sequence for encoding complementarity determining region sequences SEQ ID NO: 42, SEQ ID NO: 44 and SEQ ID NO: 46 of the light-chain variable region (VL); and a nucleotide sequence for encoding complementarity determining region sequences SEQ ID NO: 49, SEQ ID NO: 51, and SEQ ID NO: 53 of the heavy-chain variable region (VH). Due to the degeneracy of the encoding gene, the base sequence of such a polynucleotide sequence may be changed, as long as the respective corresponding complementarity determining regions may be encoded.

In a preferred technical scheme, the polynucleotide sequence includes a nucleotide sequence for encoding a sequence SEQ ID NO:4 of the light-chain variable region (VL); and a nucleotide sequence for encoding a sequence SEQ ID NO:3 of the heavy-chain variable region (VH); or the polynucleotide sequence includes a nucleotide sequence for encoding a sequence SEQ ID NO:8 of the light-chain variable region (VL); and a nucleotide sequence for encoding a sequence SEQ ID NO:7 of the heavy-chain variable region (VH); or the polynucleotide sequence includes a nucleotide sequence for encoding a sequence SEQ ID NO:12 of the light-chain variable region (VL); and a nucleotide sequence for encoding a sequence SEQ ID NO:11 of the heavy-chain variable region (VH).

In a more preferred technical scheme, the polynucleotide sequence is: SEQ ID NO: 2; or SEQ ID NO: 6; or SEQ ID NO: 10.

In an embodiment of the disclosure, a phagemid expression vector is provided, including the polynucleotide sequence of the disclosure. It is known to those skilled in the art that under the spirit of the disclosure, many vectors, such as pComb3XSS, pComb3XTT, and pComb3HSS, may be used as the expression vectors of the polynucleotide sequence of the disclosure. In a preferred embodiment, the expression vector is a phage display vector pComb3XSS (purchased from Wuhan Miaoling Biotechnology Co., Ltd).

In an embodiment of the disclosure, a host cell is provided, containing the expression vector of the disclosure, capable of expressing an anti-BCMA single-chain antibody scFv on the surface of a phage. It is known to those skilled in the art that under the spirit of the disclosure, many cells such as ER2738, SS320, TG1, XL1-Blue, ect. may be used as the host cells for the expression vectors of the disclosure. In a preferred embodiment, the host cells are an Escherichia coli XL1-Blue strain (purchased from TAKARA) and a TG1 strain (purchased from Lucigen, USA).

In an embodiment of the disclosure, a pharmaceutical composition is provided, containing the anti-BCMA single-chain antibody scFv of the disclosure, and a pharmaceutically acceptable carrier, a diluent or an excipient.

The pharmaceutical composition of the disclosure may be prepared by methods well-known in the field (for example, Remington: The Science and Practice of Pharmacy, 19th ed. (1995), A. Gennaro et al., Mack Publishing Co.), and includes the anti-BCMA single-chain antibody scFv disclosed in the disclosure and one or more pharmaceutically acceptable carriers, diluents or excipients.

In an embodiment of the disclosure, a method for preparing the anti-BCMA single-chain antibody scFv of the disclosure is provided, including transforming an expression vector containing the polynucleotide sequence of the disclosure to an expression host cell, culturing, and performing mass expression and purification of the anti-BCMA single-chain antibody scFv. In a preferred embodiment, the expression vector is a phage display vector pComb3XSS, and the host cells are an Escherichia coli XL1-Blue strain and a TG1 strain.

The anti-BCMA single-chain antibody scFv of the disclosure may be used to prepare anti-BCMA protein monoclonal antibody drugs and may also be used to immunologically detect the BCMA. Therefore, in an embodiment of the disclosure, an application of the anti-BCMA single-chain antibody scFv of the disclosure in preparing a BCMA-targeted drug, or an application in immunologically detecting BCMA for a non-disease diagnosis and treatment purpose is provided.

The technical schemes of the disclosure are described in detail below through the embodiments, and it should be understood that the embodiments are only exemplary and may not be understood as limitation to the scope of protection of the disclosure.

## Embodiment 1: Construction of phage display library for randomly synthesized single-chain antibodies

(1) Mutation design of CDR3 region

Based on heavy-chain and light-chain sequences of the humanized scFv of anti-BCMA, the mutation primers were designed respectively, and mutations were introduced in CDR3 regions of the heavy-chain and the light-chain, respectively, to construct a synthetic secondary library (for site-directed mutation) and random library (for random mutation), and mutation primer sequences are shown in Table 3.

Table 3: Primer information used for anti-BCMA antibody scFv mutation

| Library name | Primer name | Primer sequence (5'to3') (for Circle amplification of single primer) |
|---|---|---|
| Secondary library | H3_primer | GTGTATTACTGCGCCARGGSTKCTRTTTWCRACGSTT WCGAMRTTCTGGACAACTGGGGC (SEQ ID NO:55) |
| | L3_primer | TACTACTGCCAGCAGTWCARGARGMTCSCATKGACTT TCGGCCAGGGC (SEQ ID NO:56) |

(continued)

| Library name | Primer name | Primer sequence (5'to3') (for Circle amplification of single primer) |
|---|---|---|
| Random library | H3_2 | CCGCTGTGTATTACTGCGCCARGNNKNNKGSTWTKCT GGACAACTGGGGCCA (SEQ ID NO:57) |
| | H3_4 | CCGCTGTGTATTACTGCGCCARGNNKNNKNNKNNKGS TWTKCTGGACAACTGGGGCCA (SEQ ID NO:58) |
| | H3_9 | CCGCTGTGTATTACTGCGCCARGNNKNNKNNKNNKNN KNNKNNKNNKNNKGSTWTKCTGGACAACTGGGGCCA (SEQ ID NO:59) |
| | H3_11 | CCGCTGTGTATTACTGCGCCARGNNKNNKNNKNNKNN KNNKNNKNNKNNKNNKNNKGSTWTKCTGGACAACTGG GGCCA (SEQ ID NO:60) |
| | L3_4 | CCACCTACTACTGCCAGCAGNNKNNKNNKNNKCYGWT CACTTTCGGCCAGGGCAC (SEQ ID NO:61) |
| | L3_5 | CCACCTACTACTGCCAGCAGNNKNNKNNKNNKNNKCY GWTCACTTTCGGCCAGGGCAC (SEQ ID NO:62) |
| | L3_9 | CCACCTACTACTGCCAGCAGNNKNNKNNKNNKNNKNN KNNKNNKNNKCYGWTCACTTTCGGCCAGGGCAC (SEQ ID NO:63) |
| (Note: merged base K=G/T, M=A/C, R=A/G, S=G/C, W=A/T, Y=C/T) | | |

(2) Template preparation

Heavy-chain and light-chain variable region genes of the BCMA antibody were synthesized and inserted into the pComb3XSS vector (purchased from Wuhan Miaoling Biotechnology Co., Ltd) from the site Sfi I, and the primer information for antibody fragment amplification is shown in Table 4.

Table 4: Primer information used for BCMA antibody fragment amplification

| Primer name | Primer sequence (5'to3') |
|---|---|
| Primer_F | CTACCGTGGCCCAGGACATCCAGATGA (SEQ ID NO:64) |
| Primer_R | TGGTGCTGGCCGGGCTGGACACGGTCA (SEQ ID NO:65) |

1μL of a constructed template plasmid was taken, and XL1-Blue competent cells (purchased from TAKARA) were transformed, resuscitation was performed at 37°C for 1 h, 200μL was taken for coating a plate, and the coated plate was incubated at 37°C overnight.

A single clone was picked to an LB medium containing ampicillin (Amp+) M13KO7 (helper phage) (laboratory preparation), and cultured overnight at 37°C.

Bacterial solution was centrifuged to collect the supernatant, and 1/5 volume of PEG/NaCl was added and mixed uniformly, it was incubated on ice for 30 min, phage particles were precipitated, centrifugation was performed at 12,000 rpm for 30 min, the supernatant was discarded, the phage particles were resuspended with PBS, and the centrifugation was performed again at 12,000 rpm for 5 min, supernatant was collected and added to cultured CJ236 bacterial solution (purchased from TAKARA), it was used for forming dU-ssDNA (uracil-containing single-stranded template DNA), it was cultured at 37°C for 30 min, M13KO7 was added, and it was continuously cultured for 1 h, the bacterial solution was transferred to 300 ml of a medium containing Amp+, Kanamycin (Kan+) and Uridine, and it was cultured overnight at 37°C.

The bacterial solution was centrifuged, supernatant was collected, 1/4 volume of PEG/NaCl was added, and mixed uniformly, it was incubated on ice for 30 min, phage particles were precipitated, centrifugation was performed at 12000 rpm for 25 min, the supernatant was discarded, the phage particles were collected, and a DNA extraction kit was used to extract dU-ssDNA, and it was quantified.

(3) Library construction

Dry powder of each primer was prepared into 100μM of working solution, and the random library primers were mixed into heavy-chain primers H3-211 (H3-2, H3-4, H3-9, H3-11, and each 5μL) and light-chain primers L3-459 (L3-4 , L3-5, L3-9, and each 5μL).

Phosphorylation reactions were performed on each primer of the secondary library and the random library, 2 reactions per library, and 20μL per reaction system, it was as follows: primer (100 μM) 2μL, 10X T4 Buffer 2μL, T4 polynucleotide kinase 2μL, and deionized water 14μL. A water bath was performed at 37°C for 1.5h.

Phosphorylated mutation primers corresponding to the secondary library and random library were mixed respectively and annealed with the dU-ssDNA template, 1 reaction per library, and 250μL per reaction system, it was as follows: primers (each library includes light-chain and heavy-chain 2 primers) 40μL, 10X T4 Buffer 25μL, dU-ssDNA template 20μL, and deionized water supplemented to 250μL.

An annealing program was set in a PCR instrument: 90°C, 5min; 75°C, 45s; 70°C, 1min; 65°C, 1min; 60°C, 1min; 55°C, 5min; 50°C, 5min; 45°C, 30s; 37 °C, 10min; 30°C, 45s; 25°C, 45s; 22°C, 90s; 20°C, 5min; and 1 cycle.

The followings were added in each annealing reaction: 10X T4 Buffer 10μL, dNTP (each 10 mM) 25μL, DTT (100 mM) 5μL, and T7 DNA polymerase 3μL. An extension reaction was performed at 37°C for 4 h.

5μL of T4 ligase was added to each system, and a ligation reaction was performed for 2 h at a room temperature.

A ligation product was verified by DNA electrophoresis. As shown in Fig. 1, the band was significantly shifted compared to the template, indicating that the scFv was successfully constructed on the vector.

(4) Construction of phage display library

15 μL of 3M NaAc (pH 5.5) was respectively added to the secondary library and random library to adjust a pH, the ligation product was purified by a PCR Purification Kit (QIAGEN), and a purified product was quantified with Nanodrop; 1 μL of the purified secondary library and random library were respectively taken to transform high-efficiency TG competent cells (Lucigen, USA), resuscitation was performed at 37°C for 1 h, and it was gradient-diluted to $10^6$, 300 μL was respectively taken to coat a plate, it was cultured overnight at 37°C; and the number of clones on the plate was counted on the next day, according to a formula:

$$\text{Library capacity}=\text{number of clones}*\text{dilution}*1000/30$$

The library capacity of the secondary library and random library obtained by calculating was greater than $10^8$/ml. All clones were eluted with LB, centrifugation was performed at 5,000 g for 5 min, and a precipitate was suspended with 2 ml of the LB, an equal volume of 30% glycerol was added, and it was frozen at -80°C.

(5) Diversity detection of phage display library

36 clones were randomly picked from the secondary library and the random library in the step (4) respectively, and served as templates to perform clone PCR, PCR products were detected through 1.5% agarose gel electrophoresis, as shown in Fig. 2, recombination rates of the secondary library (clone 1-36) and random library (clone 37-72) were 94% and 100%. 56 clones were randomly selected and sent to Sanger sequencing to analyze the diversity of the phage display library; the results of the analysis of the scFv sequence showed that 11 of the 26 sequences in the secondary library had mutations, and 14 of the 25 sequences in the random library had mutations, the diversity reached 42% and 56% respectively, it may be used for the next step of screening of new antibodies.

(6) Phage amplification and rescue

Proliferation and phage rescue were performed on the phage display of the secondary library and the random library obtained above, 1 ml of the phage library stored in the step (4) was respectively inoculated in 100 ml of a medium and cultured to a logarithmic growth phase, and a helper phage M13K01 of which MOI (multiplicity of infection) was 20 was added, it was standing for 30 min at a room temperature, after low-speed centrifugation, the precipitate was suspended with a medium, inoculated in 300 ml medium, and cultured overnight. On the next day, centrifugation was performed for 30 min at 3,000, supernatant was collected, and PEG was added to precipitate the phage, it was standing on ice for 30 min, the centrifugation was performed at 3,000 for 30 min, and the precipitate was the phage library of the scFv carrying the BCMA, after the precipitate was suspended with PBS, a titer thereof was measured, the secondary library was 2.6 X $10^{12}$ pfu/ml, and the random library was 3.3 X $10^{12}$ pfu/ml.

Embodiment 2: High-affinity antibodies of BCMA screened with phage display technology

(1) Phage affinity panning

100 ng of a BCMA-his antigen-coated ELISA plate was respectively taken as a sample well, 100 $\mu$L of $Na_2CO_3$ was served as a negative control well, and it was incubated overnight at 4°C. On the next day, the phage libraries of the secondary library and random library rescued in the step (6) of Embodiment 1 were added respectively, and incubated at a room temperature for 2 h; the wells were washed with PBST for 10 times, 100 $\mu$L of triethylamine was added, and incubated at a room temperature for 30 min, the collected phage was the phage library of the anti-BCMA scFv obtained by affinity panning; 10 $\mu$L of infected TG cells were taken to coat a plate, the biopanning result of the affinity phage was observed on the next day, and the remaining phages were used for amplification and rescue.

(2) Amplification and rescue of phage after biopanning

Amplification and rescue methods were the same as the step (6) in Embodiment 1, the obtained PBS suspension, which was the amplified phage after the first round of biopanning, is stored at 4°C and used for the next round of screening; and according to the biopanning step of the step (1) in Embodiment 2, an amount of the antigen was gradually decreased in each round, and the biopanning was performed for 3-4 rounds.

(3) Enrichment degree of specific antibodies evaluated by ELISA

100 ng of BCMA-his antigen was taken, and placed at 4°C overnight; on the next day, 2% BSA was added, and blocked for 1 h at a room temperature; the phage amplified after each round of the biopanning was respectively added in an experimental group, and the same amount of a wild-type phage was added in a control group, and incubated at a room temperature for 2 h; it was washed with PBST for 10 times to remove unbound phages; a HRP-labeled anti-M13 antibody was added, and incubated at a room temperature for 1 h; color developing solution was added, and reacted for 10-30 min in the dark, an absorbance value $OD_{450}$ was measured, and the ELISA result was shown in Fig. 3, the absorbance value tended to be stable from the first to the third round of the biopanning, indicating that the new specific antibodies were enriched.

(4) Identification of BCMA-targeted high-affinity scFv positive clones

The phage-coated plates obtained in the second and third rounds of biopanning were taken, and 36 and 20 clones from the random library and the secondary library were selected respectively for clone PCR; the results were shown in Fig. 4, and clones with shifted band were selected for sequencing; the sequencing results were analyzed, and it was discovered that there were 5 scFv monoclonal strains (indicated by arrows in the figure) with mutations in the CDR3 regions.

The ELISA plate was coated with 100 ng BCMA-Fc antigen, and incubated overnight at 4°C; 5 single clones were picked and placed in 1 ml medium, cultured at 37°C to the logarithmic phase, and induced overnight by adding 1 mM IPTG; on the next day, the bacterial precipitate was collected by centrifugation, after crushed, centrifuged at 5,000 g for 15 min, and supernatant was collected; at the same time, the ELISA plate was taken, 2% of BSA was added and blocked for 1 h at a room temperature; monoclonal crushed supernatant was added to each well of the experimental group, and blank TG crushed supernatant was added to the control group, and incubated at a room temperature for 2 h; it was washed with PBST for 10 times, a mouse anti-HA-tag antibody was added, and placed at a room temperature for 1 h; it was washed with the PBST for 3-5 times, an AP-labeled anti-mouse IgG antibody was added, and placed at a room temperature for 1 h; the substrate was added, and reacted for 10-20 min, an absorbance value was read in a microplate reader; while a ratio of the absorbance value and the control well was greater than 2.1 (baseline), it was judged to be a positive clone; and an ELISA verification result showed that 3 positive clones were obtained, namely a clone 20, a clone 43 and a clone 46 (Fig. 5).

(5) Sequence information of positive clones

The 3 positive clones obtained in the step (4) were compared with the original sequence, it was discovered that they had significant mutations in the CDR3 region of the heavy-chain and the CDR3 region of the light-chain. The 3 scFv monoclonal strains were respectively named as scFv_20, scFv_43, and scFv_46, and nucleotide and amino acid sequences thereof are as follows:

scFv_20 nucleotide sequence:

5'-GACATCCAGATGACCCAGAGCCCTAGCTCACTGAGCGCCAGCGTGGGCGACAGGGTGACCATTACCTGCTCCGCCAGCCAGGACATCAGCAACTACCTGAACTGGTACCAGCAGAAGCCCGGCAAGGCCCCCAAGCTGCTGATCTACTACACCTCCAACCTGCACTCCGGCGTGCCCAGCAGGTTCAGCGGAAGCGGCAGCGGCACCGATTTCACCCTGACCATCTCCAGCCTGCAGCCCGAGGACTTCGCCACCTACTACTGCCAGCAGTACAGGAAGCTCGCATGGACTTTCGGCCAGGGCACCAAACTGGAGATCAAGCGTGGTGGAGGAGGTAGCGGAGGAGGCGGGAGCGGTGGAGGTGGCTCTGGAGGTGGCGGAAGCCAGGTGCAGCTGGTCCAGAGCGGCGCCGAAGTGAAGAAGCCCGGCAGCTCCGTGAAAGTGAGCTGCAAGGCCAGCGGCGGCACCTTCAGCAACTACTGGATGCACTGGGTGAGGCAGGCCCCCGGACAGGGCCTGGAGTGGATGGGCGCCACCTACAGGGGCCACAGCGACACCTACTACAACCAGAAGTTCAAGGGCCGGGTGACCATCACCGCCGACAAGAGCACCAGCACCGCCTACATGGAACTGAGCAGCCTCAGGAGCGAGGACACCGCTGTGTATTACTGCGCCAGGGGTTCTATTTTCAACGGTTACGACGTTCTGGACAACTGGGGCCAGGGCACACTAGTGACCGTGTCCAGC-3' (SEQ ID NO:2).

scFv_20 amino acid sequence:

DIQMTQSPSSLSASVGDRVTITCSASQDISNYLNWYQQKPGKAPKLLIYYTSNLHSGVPSRFSGSGSGTDFTLTISSLQPEDFATYYCQQYRKLAWTFGQGTKLEIKRGGGGSGGGGSGGGGSGGGGSQVQLVQSGAEVKKPGSSVKVSCKASGGTFSNYWMHWVRQAPGQGLEWMGATYRGHSDTYYNQKFKGRVTITADKSTSTAYMELSSLRSEDTAVYYCARGSIFNGYDVLDNWGQGTLVTVSS (SEQ ID NO:1).

scFv_43 nucleotide sequence:

5'-GACATCCAGATGACCCAGAGCCCTAGCTCACTGAGCGCCAGCGTGGGCGACAGGGTG ACCATTACCTGCTCCGCCAGCCAGGACATCAGCAACTACCTGAACTGGTACCAGCAGAAGCCC GGCAAGGCCCCCAAGCTGCTGATCTACTACACCTCCAACCTGCACTCCGGCGTGCCCAGCAG GTTCAGCGGAAGCGGCAGCGGCACCGATTTCACCCTGACCATCTCCAGCCTGCAGCCCGAG GACTTCGCCACCTACTACTGCCAGCAGGCTCTTGTGGTGACGCCGTTCACTTTCGGCCAGGG CACCAAACTGGAGATCAAGCGTGGTGGAGGAGGTAGCGGAGGAGGCGGGAGCGGTGGAGG TGGCTCTGGAGGTGGCGGAAGCCAGGTGCAGCTGGTCCAGAGCGGCGCCGAAGTGAAGAA GCCCGGCAGCTCCGTGAAAGTGAGCTGCAAGGCCAGCGGCGGCACCTTCAGCAACTACTGG ATGCACTGGGTGAGGCAGGCCCCCGGACAGGGCCTGGAGTGGATGGGCGCCACCTACAGG GGCCACAGCGACACCTACTACAACCAGAAGTTCAAGGGCCGGGTGACCATCACCGCCGACAA GAGCACCAGCACCGCCTACATGGAACTGAGCAGCCTCAGGAGCGAGGACACCGCTGTGTATT ACTGCGCCAGGTTTTAGGGTATGCTGGACAACTGGGGCCAGGGCACACTAGTGACCGTGTCC AGC-3' (SEQ ID NO:6).

scFv_43 amino acid sequence:

DIQMTQSPSSLSASVGDRVTITCSASQDISNYLNWYQQKPGKAPKLLIYYTSNLHSGVPSRFS GSGSGTDFTLTISSLQPEDFATYYCQQALVVTPFTFGQGTKLEIKRGGGGSGGGGSGGGGSGGG GSQVQLVQSGAEVKKPGSSVKVSCKASGGTFSNYWMHWVRQAPGQGLEWMGATYRGHSDTY YNQKFKGRVTITADKSTSTAYMELSSLRSEDTAVYYCARFGMLDNWGQGTLVTVSS (SEQ ID NO:5).

scFv_46 nucleotide sequence:

5'-GACATCCAGATGACCCAGAGCCCTAGCTCACTGAGCGCCAGCGTGGGCGACAGGGTG

ACCATTACCTGCTCCGCCAGCCAGGACATCAGCAACTACCTGAACTGGTACCAGCAGAAGCCC GGCAAGGCCCCCAAGCTGCTGATCTACTACACCTCCAACCTGCACTCCGGCGTGCCCAGCAG GTTCAGCGGAAGCGGCAGCGGCACCGATTTCACCCTGACCATCTCCAGCCTGCAGCCCGAG GACTTCGCCACCTACTACTGCCAGCAGCGTTTGACGCCGTCTCCGTTCACTTTCGGCCAGGG CACCAAACTGGAGATCAAGCGTGGTGGAGGAGGTAGCGGAGGAGGCGGGAGCGGTGGAGG TGGCTCTGGAGGTGGCGGAAGCCAGGTGCAGCTGGTCCAGAGCGGCGCCGAAGTGAAGAA GCCCGGCAGCTCCGTGAAAGTGAGCTGCAAGGCCAGCGGCGGCACCTTCAGCAACTACTGG ATGCACTGGGTGAGGCAGGCCCCCGGACAGGGCCTGGAGTGGATGGGCGCCACCTACAGG GGCCACAGCGACACCTACTACAACCAGAAGTTCAAGGGCCGGGTGACCATCACCGCCGACAA GAGCACCAGCACCGCCTACATGGAACTGAGCAGCCTCAGGAGCGAGGACACCGCTGTGTATT ACTGCGCCAGGAATACGGCTTTGCTGGACAACTGGGGCCAGGGCACACTAGTGACCGTGTCC AGC-3' (SEQ ID NO:10).

scFv_46 amino acid sequence:

DIQMTQSPSSLSASVGDRVTITCSASQDISNYLNWYQQKPGKAPKLLIYYTSNLHSGVPSRFS GSGSGTDFTLTISSLQPEDFATYYCQQRLTPSPFTFGQGTKLEIKRGGGGSGGGGSGGGGSGGG GSQVQLVQSGAEVKKPGSSVKVSCKASGGTFSNYWMHWVRQAPGQGLEWMGATYRGHSDTY YNQKFKGRVTITADKSTSTAYMELSSLRSEDTAVYYCARNTALLDNWGQGTLVTVSS (SEQ ID NO:9).

The disclosure is described by using specific examples above, which are only used to facilitate the understanding of the disclosure, and not used to limit the disclosure. For those skilled in the art of the disclosure, a plurality of simple deductions, modifications or replacements may also be made according to ideas of the disclosure.

## Claims

**1.** An anti-BCMA single-chain antibody scFv, wherein the single-chain antibody scFv comprises a light-chain variable region (VL) and a heavy-chain variable region (VH),

the light-chain variable region (VL) comprises a framework region (FR) and a complementarity determining region (CDR), wherein the complementarity determining region (CDR) comprises a CDR1, wherein a sequence thereof is QDISNY (SEQ ID NO:14), a CDR2, wherein a sequence thereof is YTS (SEQ ID NO:16), and a CDR3, wherein a sequence thereof is QQYRKLAWT (SEQ ID NO:18); and the heavy-chain variable region (VH) comprises a framework region (FR) and a complementarity determining region (CDR), wherein the complementarity determining region (CDR) comprises a CDR1, wherein a sequence thereof is GGTFSNYW (SEQ ID NO:21), a CDR2, wherein a sequence thereof is TYRGHSDT (SEQ ID NO:23), and a CDR3, wherein a sequence thereof is ARGAIYNGYDVLDN (SEQ ID NO:25); or

the light-chain variable region (VL) comprises a framework region (FR) and a complementarity determining region (CDR), wherein the complementarity determining region (CDR) comprises a CDR1, wherein a sequence thereof is QDISNY (SEQ ID NO:28), a CDR2, wherein a sequence thereof is YTS (SEQ ID NO:30), and a CDR3, wherein a sequence thereof is QQALVVTPFT (SEQ ID NO:32); and the heavy-chain variable region (VH) comprises a framework region (FR) and a complementarity determining region (CDR), wherein the complementarity determining region (CDR) comprises a CDR1, wherein a sequence thereof is GGTFSNYW (SEQ ID NO:35), a CDR2, wherein a sequence thereof is TYRGHSDT (SEQ ID NO:37), and a CDR3, wherein a sequence thereof is ARGAIYNGYDVLDN (SEQ ID NO:39); or

the light-chain variable region (VL) comprises a framework region (FR) and a complementarity determining region (CDR), wherein the complementarity determining region (CDR) comprises a CDR1, wherein a sequence thereof is QDISNY (SEQ ID NO:42), a CDR2, wherein a sequence thereof is YTS (SEQ ID NO:44), and a CDR3, wherein a sequence thereof is QQRLTPSPFT (SEQ ID NO:46); and the heavy-chain variable region (VH) comprises a framework region (FR) and a complementarity determining region (CDR), wherein the complementarity determining region (CDR) comprises a CDR1, wherein a sequence thereof is GGTFSNYW (SEQ ID NO:49), a CDR2, wherein a sequence thereof is TYRGHSDT (SEQ ID NO:51), and a CDR3, wherein a sequence thereof is ARNTALLDN (SEQ ID NO:53).

**2.** The single-chain antibody scFv as claimed in claim 1, wherein a sequence of the light-chain variable region (VL) is SEQ ID NO:4, and a sequence of the heavy-chain variable region (VH) is SEQ ID NO:3; or

a sequence of the light-chain variable region (VL) is SEQ ID NO:8, and a sequence of the heavy-chain variable region (VH) is SEQ ID NO:7; or
a sequence of the light-chain variable region (VL) is SEQ ID NO:12, and a sequence of the heavy-chain variable region (VH) is SEQ ID NO:11.

**3.** The single-chain antibody scFv as claimed in claim 1, wherein a sequence of the single-chain antibody scFv is SEQ ID NO:1; or SEQ ID NO:5; or SEQ ID NO:9.

**4.** A polynucleotide sequence for encoding the anti-BCMA single-chain antibody scFv as claimed in any one of claims 1-3, wherein the polynucleotide sequence comprises a nucleotide sequence for encoding complementarity determining region sequences SEQ ID NO: 14, SEQ ID NO: 16 and SEQ ID NO: 18 of the light-chain variable region (VL); and a nucleotide sequence for encoding complementarity determining region sequences SEQ ID NO: 21, SEQ ID NO: 23, and SEQ ID NO: 25 of the heavy-chain variable region (VH); or

the polynucleotide sequence comprises a nucleotide sequence for encoding complementarity determining region sequences SEQ ID NO: 28, SEQ ID NO: 30 and SEQ ID NO: 32 of the light-chain variable region (VL); and a nucleotide sequence for encoding complementarity determining region sequences SEQ ID NO: 35, SEQ ID NO: 37, and SEQ ID NO: 39 of the heavy-chain variable region (VH); or
the polynucleotide sequence comprises a nucleotide sequence for encoding complementarity determining region sequences SEQ ID NO: 42, SEQ ID NO: 44 and SEQ ID NO: 46 of the light-chain variable region (VL); and a nucleotide sequence for encoding complementarity determining region sequences SEQ ID NO: 49, SEQ ID NO: 51, and SEQ ID NO: 53 of the heavy-chain variable region (VH).

**5.** The polynucleotide sequence as claimed in claim 4, wherein the polynucleotide sequence comprises a nucleotide sequence for encoding a sequence SEQ ID NO:4 of the light-chain variable region (VL); and a nucleotide sequence for encoding a sequence SEQ ID NO:3 of the heavy-chain variable region (VH); or

the polynucleotide sequence comprises a nucleotide sequence for encoding a sequence SEQ ID NO:8 of the light-chain variable region (VL); and a nucleotide sequence for encoding a sequence SEQ ID NO:7 of the heavy-chain variable region (VH); or
the polynucleotide sequence comprises a nucleotide sequence for encoding a sequence SEQ ID NO:12 of the light-chain variable region (VL); and a nucleotide sequence for encoding a sequence SEQ ID NO:11 of the heavy-chain variable region (VH).

**6.** The polynucleotide sequence as claimed in claim 4, wherein the polynucleotide sequence is SEQ ID NO: 2; or SEQ ID NO: 6; or SEQ ID NO: 10.

**7.** An expression vector, wherein the expression vector comprises the polynucleotide sequence as claimed in any one of claims 3-6.

**8.** A host cell, wherein the host cell comprises the expression vector as claimed in claim 7, and can express an anti-BCMA single-chain antibody scFv.

**9.** A pharmaceutical composition, wherein the pharmaceutical composition comprises the anti-BCMA single-chain antibody scFv as claimed in any one of claims 1-3, and a pharmaceutically acceptable carrier, a diluent or a excipient.

**10.** A method for preparing the anti-BCMA single-chain antibody scFv as claimed in any one of claims 1-3, wherein the method comprises: transforming an expression vector containing the polynucleotide sequence as claimed in any one of claims 3-6 to an expression host cell, culturing, and performing mass expression and purification of the anti-BCMA single-chain antibody scFv.

**11.** The method as claimed in claim 10, wherein the expression vector is a pComb3XSS vector, and the host cells are Escherichia coli XL1-Blue and a TG1 strain.

**12.** Use of the anti-BCMA single-chain antibody scFv as claimed in any one of claims 1-3 in preparing a BCMA-targeted drug, or in immunologically detecting BCMA for a non-disease diagnosis and treatment purpose.

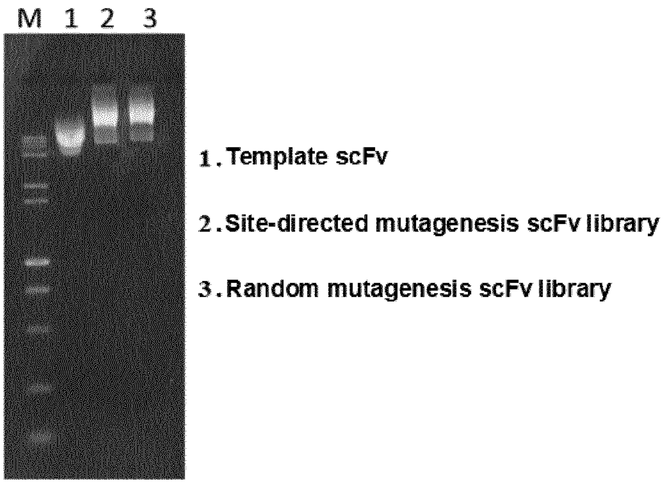
M 1 2 3
1. Template scFv
2. Site-directed mutagenesis scFv library
3. Random mutagenesis scFv library

Fig. 1

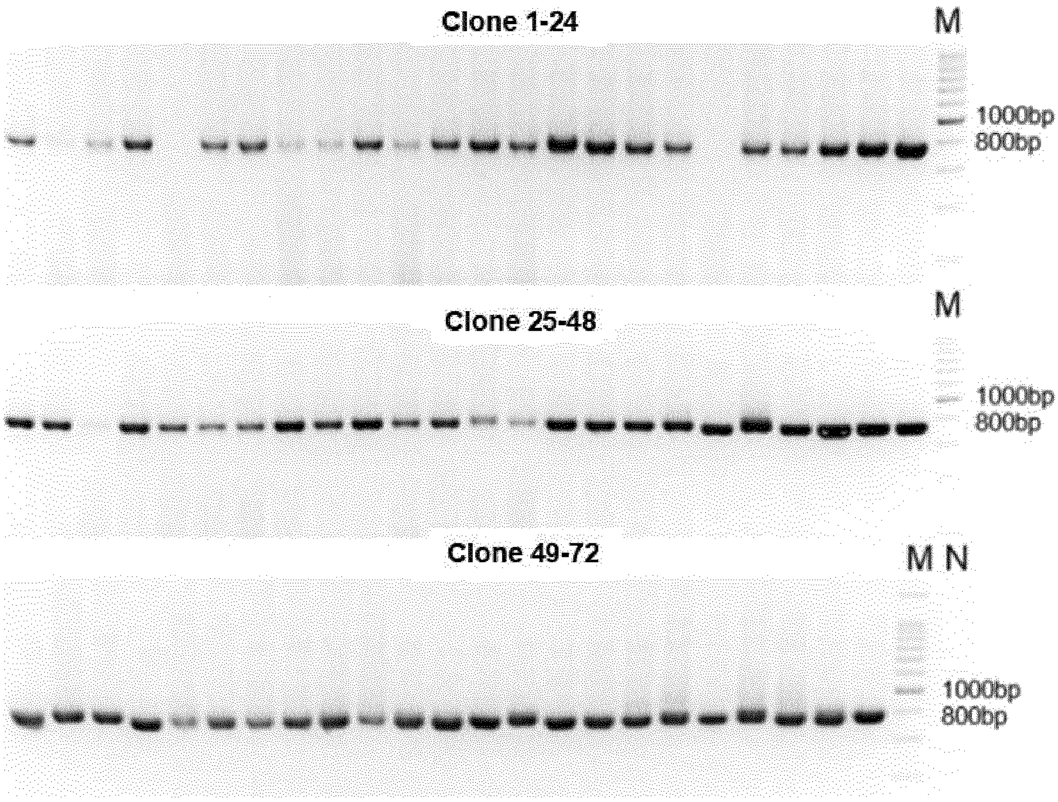
Clone 1-24
M
1000bp
800bp
Clone 25-48
M
1000bp
800bp
Clone 49-72
M N
1000bp
800bp

Fig. 2

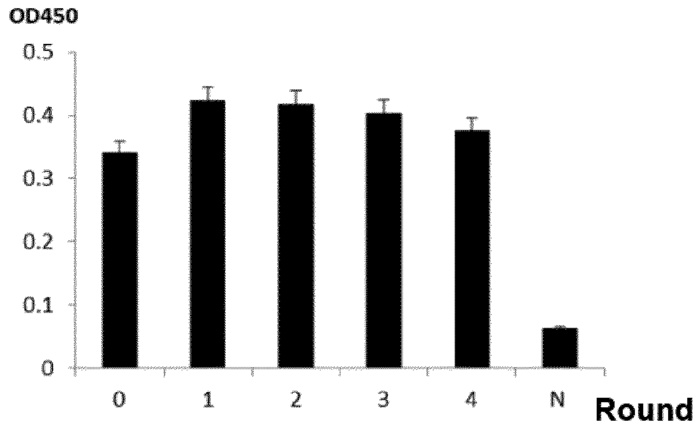
OD450
0.5
0.4
0.3
0.2
0.1
0
0
1
2
3
4
N
Round

Fig. 3

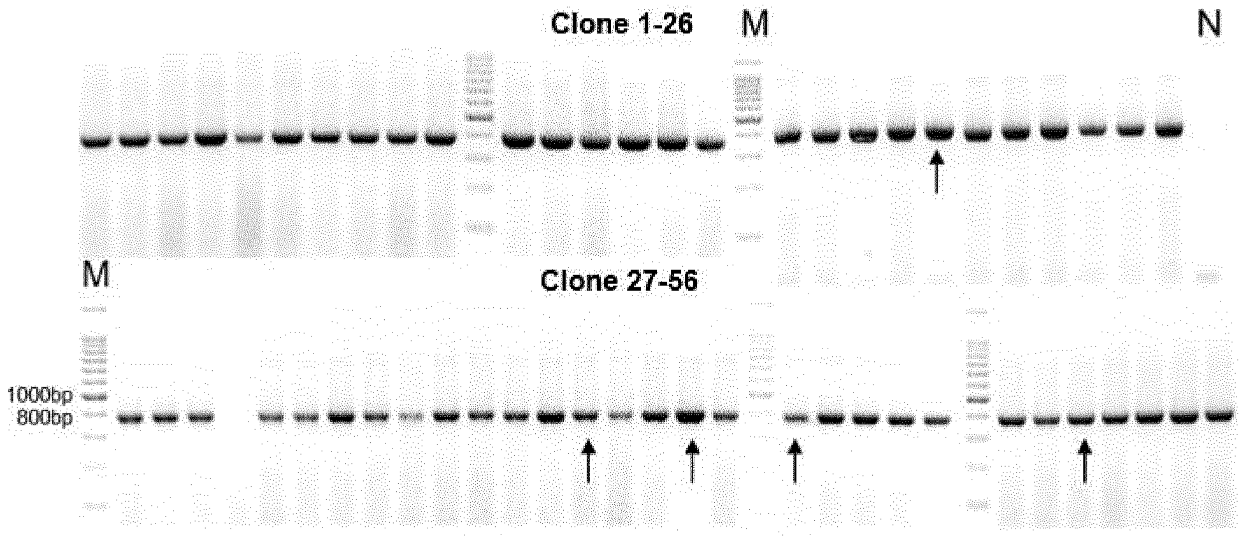
Clone 1-26
M
N
M
Clone 27-56
1000bp
800bp

Fig. 4

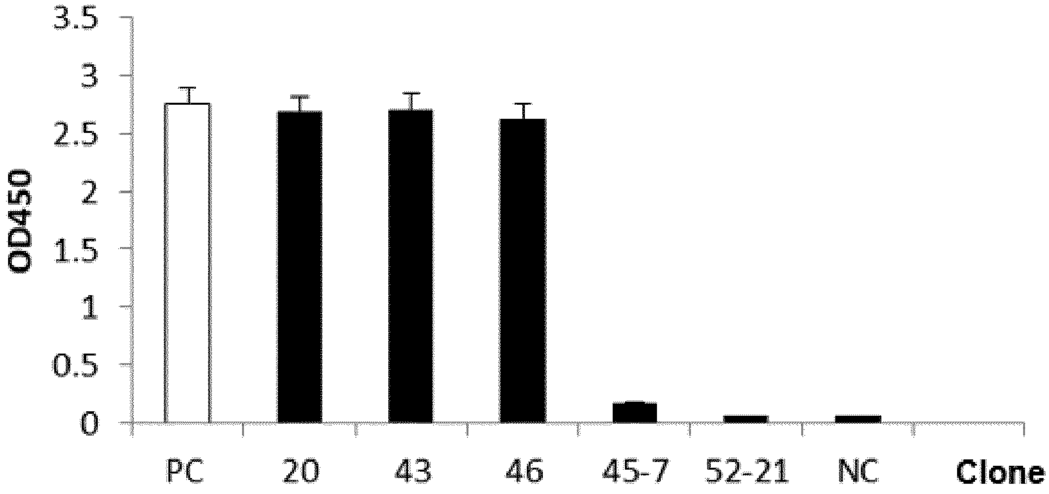


Fig. 5

**INTERNATIONAL SEARCH REPORT**

International application No. **PCT/CN2018/109564**

**A. CLASSIFICATION OF SUBJECT MATTER**

C07K 16/28(2006.01)i; C12N 15/62(2006.01)i; C12N 5/10(2006.01)i; A61K 35/17(2015.01)i; A61P 35/00(2006.01)i

According to International Patent Classification (IPC) or to both national classification and IPC

**B. FIELDS SEARCHED**

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNABS, CNKI, CNTXT, DWPI, SIPOABS, EPTXT, USTXT, WOTXT, JPTXT, NCBI, 中国专利生物序列检索系统, Genbank, EMBL, STN和检索项：B细胞成熟抗原, BCMA, BMCA, 抗体, 单链抗体, scFv, SEQ ID NO.1

**C. DOCUMENTS CONSIDERED TO BE RELEVANT**

| Category* | Citation of document, with indication, where appropriate, of the relevant passages | Relevant to claim No. |
|---|---|---|
| A | CN 106687483 A (NOVARTIS AG; UNIVERSITY OF PENNSYLVANIA) 17 May 2017 (2017-05-17)<br>claims 8, 44, description paragraphs 1207, 1235 | 1-12 |
| A | CN 107208047 A (MEMORIAL SLOAN-KETTERING CANCER CENTER; EUREKA THERAPEUTICS INC) 26 September 2017 (2017-09-26)<br>claims 5, 81, 109 | 1-12 |
| A | CN 108473575 A (US HEALTH; SANFORD RESEARCH) 31 August 2018 (2018-08-31)<br>entire document | 1-12 |
| A | CN 105837693 A (LI, Siwen; ZHAO, Lei) 10 August 2016 (2016-08-10)<br>claims 1-10 | 1-12 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 May 2019** | **17 June 2019** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

INTERNATIONAL SEARCH REPORT

International application No.
PCT/CN2018/109564

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2018/109564**

**Box No. III Observations where unity of invention is lacking (Continuation of item 3 of first sheet)**

This International Searching Authority found multiple inventions in this international application, as follows:

[1] Invention 1: claims 1-13 (in part);

[2] relating to an anti-BCMA single-chain antibody variable fragment (scFv), the sequence of the scFv being SEQ ID NO: 1, and the corresponding light chain variable region and heavy chain variable region, polynucleotide, expression vector, host cell, pharmaceutical composition, preparation method, and use in preparing a BCMA-targeting medicament.

[3] Inventions 2 and 3: claims 1-13 (in part); the subject matter is the same as that of invention 1, but the amino acid sequences to which claims 2 and 3 relate are as follows:

[4] invention 2: SEQ ID NO: 5

[5] invention 3: SEQ ID NO: 9

[6] The corresponding technical feature in claims 1-3, i.e. "an anti-BCMA single-chain antibody variable fragment (scFv)", is disclosed in the prior art (CN 107208047 A, 26 September 2017, see claim 5). Therefore, the claimed technical solutions of claims 1-13 do not fall within a single general inventive concept, are not technically linked to each other, do not have a same or corresponding special technical feature, do not comply with the unity, and do not comply with PCT Rule 13.1 and 13.2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **1-3 (relating to an anti-BCMA single-chain antibody variable fragment (scFv), the sequence of said scFv being SEQ ID NO: 1)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2018/109564**

| Patent document cited in search report | | | Publication date (day/month/year) | Patent family member(s) | | | Publication date (day/month/year) |
|---|---|---|---|---|---|---|---|
| CN | 106687483 | A | 17 May 2017 | US | 10174095 | B2 | 08 January 2019 |
| | | | | EP | 3172237 | A2 | 31 May 2017 |
| | | | | RU | 2017105065 | A3 | 18 February 2019 |
| | | | | WO | 2016014565 | A3 | 10 March 2016 |
| | | | | WO | 2016014565 | A2 | 28 January 2016 |
| | | | | US | 2016046724 | A1 | 18 February 2016 |
| | | | | TW | 201615832 | A | 01 May 2016 |
| | | | | CA | 2955386 | A1 | 28 January 2016 |
| | | | | AU | 2015292744 | A1 | 02 February 2017 |
| | | | | MX | 2017001011 | A | 28 May 2018 |
| | | | | BR | 112017001183 | A2 | 28 November 2017 |
| | | | | JP | 2017527271 | A | 21 September 2017 |
| | | | | SG | 11201700476V | A | 27 February 2017 |
| | | | | KR | 20170037626 | A | 04 April 2017 |
| | | | | AU | 2015292744 | A8 | 09 March 2017 |
| | | | | RU | 2017105065 | A | 21 August 2018 |
| CN | 107208047 | A | 26 September 2017 | EP | 3227432 | A1 | 11 October 2017 |
| | | | | IL | 252617 | D0 | 31 July 2017 |
| | | | | BR | 112017011909 | A2 | 27 February 2018 |
| | | | | US | 2018360880 | A1 | 20 December 2018 |
| | | | | CA | 2969870 | A1 | 09 June 2016 |
| | | | | MX | 2017007244 | A | 25 January 2018 |
| | | | | EP | 3227432 | A4 | 22 August 2018 |
| | | | | KR | 20170109538 | A | 29 September 2017 |
| | | | | JP | 2017537925 | A | 21 December 2017 |
| | | | | SG | 10201900931 X | A | 27 February 2019 |
| | | | | SG | 11201704549 U | A | 28 July 2017 |
| | | | | RU | 2017123548 | A | 14 January 2019 |
| | | | | PH | 12017501040 | A1 | 05 March 2018 |
| | | | | WO | 2016090320 | A1 | 09 June 2016 |
| | | | | AU | 2015357526 | A1 | 29 June 2017 |
| CN | 108473575 | A | 31 August 2018 | CA | 3005042 | A1 | 18 May 2017 |
| | | | | WO | 2017083511 | A1 | 18 May 2017 |
| | | | | KR | 20190008171 | A | 23 January 2019 |
| | | | | JP | 2019505564 | A | 28 February 2019 |
| | | | | EP | 3374396 | A1 | 19 September 2018 |
| | | | | AU | 2016353067 | A1 | 24 May 2018 |
| | | | | US | 2018318435 | A1 | 08 November 2018 |
| CN | 105837693 | A | 10 August 2016 | | None | | |

Form PCT/ISA/210 (patent family annex) (January 2015)